(19) Europäisches Patentamt
European Patent Office
Office européen des brevets

(11) Publication number : **0 480 745 A2**

(12) # EUROPEAN PATENT APPLICATION

(21) Application number : **91309372.0**

(22) Date of filing : **11.10.91**

(51) Int. Cl.$^5$ : **C07D 495/04,** A61K 31/38, // (C07D495/04, 333:00, 333:00)

(30) Priority : **12.10.90 US 596188**
**04.10.91 US 769055**

(43) Date of publication of application :
**15.04.92 Bulletin 92/16**

(84) Designated Contracting States :
**AT BE CH DE DK ES FR GB IT LI LU NL SE**

(71) Applicant : **MERCK & CO. INC.**
**126, East Lincoln Avenue P.O. Box 2000**
**Rahway New Jersey 07065-0900 (US)**

(72) Inventor : **Baldwin, John J.**
**621 Gypsy Hill Circle**
**Gwynedd Valley, PA 19437 (US)**
Inventor : **Selnick, Harold G.**
**1232 Lois Road**
**Ambler, PA 19002 (US)**
Inventor : **Ponticello, Gerald S.**
**2045 Spring Valley Road**
**Lansdale, PA 19446 (US)**
Inventor : **Radzilowski, Ellen M.**
**11 Hastings Drive**
**Blue Bell, PA 19422 (US)**

(74) Representative : **Thompson, John Dr. et al**
**Merck & Co., Inc. European Patent**
**Department Terlings Park Eastwick Road**
**Harlow, Essex CM20 2QR (GB)**

(54) **Substituted dihydrothieno-thiophene-2-sulfonamides and dioxides thereof.**

(57) Substituted 4,5-dihydrothieno[2,3-b]thiophene-2-sulfonamides and 6,6-oxides and dioxides thereof and the isomeric 5,6-dihydrothieno[3,2-b]thiophene-2-sulfonamides and 4,4-dioxides thereof are topically effective carbonic anhydrase inhibitors useful in the treatment of ocular hypertension and glaucoma associated therewith.

EP 0 480 745 A2

## SUMMARY OF THE INVENTION

This invention relates to novel derivatives of dihydrothieno[2,3-b] and [3,2-b]thiophene-2-sulfonamides and their 6,6-oxides and dioxides of structural formulae I and II:

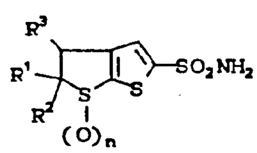

I

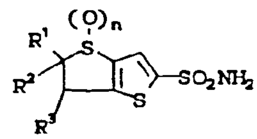

II

or an ophthalmologically acceptable salt thereof wherein n is 0, 1 or 2, and $R^1$, $R^2$, and $R^3$ are as hereinafter defined.

This invention also relates to ophthalmic formulations comprising at least one of the novel compounds as active ingredient either alone or in combination with other ophthalmic medicaments such as pilocarpine, timolol or enaliprilat,

The invention also relates to a method of treating ocular hypertension and glaucoma associated therewith which comprises the topical ocular administration of a novel compound of this invention to a patient in need of such treatment.

## BACKGROUND OF THE INVENTION

Glaucoma is an ocular disorder associated with elevated intraocular pressures which are too high for normal function and may result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Indeed, few advances were made in the treatment of glaucoma since pilocarpine and physostigmine were introduced. Only recently have clinicians noted that many β-adrenergic blocking agents are effective in reducing intraocular pressure. While many of these agents are effective in reducing intraocular pressure, they also have other characteristics, e.g. membrane stabilizing activity, that are not acceptable for chronic ocular use. (S)-1-tert-Butylamino--[(4-morpholino-1,2,5-thiadiazo1-3-yl)oxy]-2-propanol, a β-adrenergic blocking agent, was found to reduce intraocular pressure and to be devoid of many unwanted side effects associated with pilocarpine and, in addition, to possess advantages over many other β-adrenergic blocking agents, e.g. to be devoid of local anesthetic properties, to have a long duration of activity, and to display minimal tolerance.

Although pilocarpine, physostigmine and the β-blocking agents mentioned above reduce intraocular pressure, none of these drugs manifests its action by inhibiting the enzyme carbonic anhydrase and, thereby, impeding the contribution to aqueous humor formation made by the carbonic anhydrase pathway.

Agents referred to as carbonic anhydrase inhibitors block or impede this inflow pathway by inhibiting the enzyme, carbonic anhydrase. While such carbonic anhydrase inhibitors are now used to treat intraocular pressure by oral, intravenous or other systemic routes, they thereby have the distinct disadvantage of inhibiting carbonic anhydrase through-out the entire body. Such a gross disruption of a basic enzyme system is justified only during an acute attack of alarmingly elevated intraocular pressure, or when no other agent is effective. Despite the desirability of directing the carbonic anhydrase inhibitor only to the desired ophthalmic target tissue, no topically effective carbonic anhydrase inhibitors are available for clinical use.

However, topically effective carbonic anhydrase inhibitors are reported in U.S. patents 4,386,098; 4,416,890; and 4,426,388. The compounds reported therein are 5 (and 6)-hydroxy-2-benzothiazolesulfonamides and acyl esters thereof. Benzothiophene-2-sulfonamides, benzenesulfonylthiophene-2-sulfonamides, and thieno[2,3-b]thiopyran-2-sulfonamides are also reported to be carbonic anhydrase inhibitors topically effective in reducing intraocular pressure in U.S. Patents 4,668,697; 4,585,787; and 4,797,413 respectively.

## DETAILED DESCRIPTION OF THE INVENTION

The novel compounds of this invention have structural formula:

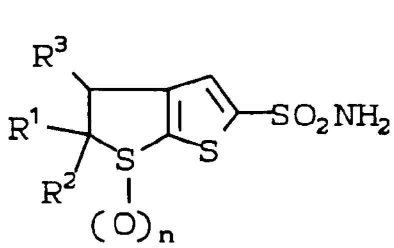

I

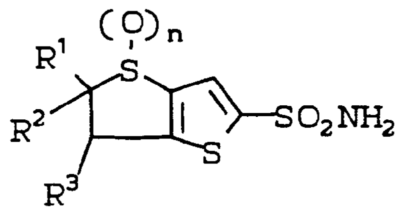

II

or the (R) or (S) enantiomer or mixtures thereof, or an ophthalmologically acceptable salt thereof wherein:
n is 0, 1 or 2, preferably 2;
$R^1$ is
1) hydrogen,
2) $C_{1-6}$alkyl, or
3) $C_{2-6}$alkenyl;
$R^2$ is
$C_{1-6}$alkyl, either unsubstituted or substituted with
a)

$C_{1-3}$alkyl-O-,
(O)$_m$

b) $C_{1-3}$alkyl-S-,
c)

$C_{1-3}$alkyl-O-$C_{1-3}$alkyl-O-
(O)$_m$

d)

$C_{1-3}$alkyl-O-$C_{1-3}$alkyl-S-,
(O)$_m$

e) $C_{1-3}$alkyl-S-$C_{1-3}$alkyl-O-,
f) mono- or di-$C_{1-3}$alkyl-O-,
g) $C_2$-$C_5$ alkenyl,
h) hydroxy,
i)

hydroxy$C_{1-3}$alkyl-O-,
(O)$_m$

j) hydroxy$C_{1-3}$alkyl-S-,
k)

hydroxy$C_{1-3}$alkyl-O-$C_{1-3}$alkyl-O-,
(O)$_m$

l) hydroxy$C_{1-3}$alkyl-O-$C_{1-3}$alkyl-S-,
m) hydroxymono- or di-$C_{1-3}$alkyl-NH-,

n) -COOH,
o) -C=N or
p) $-CONH_2$,
wherein m is 0, 1 or 2;
$R^3$
is hydroxy, amino, azido, $C_{1-6}$alkylamino, or $C_{2-6}$alkenylamino, with the alkyl group optionally substituted with hydroxy, F, $C_{1-3}$alkyl-O-, or $C_{1-3}$alkyl-$S(O)_m$-,
wherein m is 0, 1 or 2.

The preferred isomer of the novel compounds is that designated as structure I, being generically 4,5-dihydrothieno[2,3-b]thiophene-2-sulfonamides.

It is also preferred that $R^2$ be $C_{1-6}$alkyl, $C_{1-3}$alkoxy-$C_{1-6}$alkyl and $C_{1-3}$alkoxy $C_{1-3}$alkoxy alkyl and that $R^3$ be $C_{1-6}$alkylamino.

The terms "alkyl" and "alkoxy" as used herein are meant to include: straight chain alkyl and alkoxy; branched chain alkyl and alkoxy; and $C_{1-6}$alkyl and $C_{2-6}$alkyl includes $C_{3-6}$cycloalkyl.

These novel compounds include both the racemic and enantiomerically pure diastereomers or mixtures thereof.

The novel process of this invention is illustrated by the following reaction scheme. The designations "α" and "β" refer to the chromatographic mobility of the two isomers. The α compounds elutes faster than the β.

A general process for preparation of a key intermediate, IX, in the preparation of the novel compounds of structure I is depicted in Reaction Scheme I. This Reaction Scheme and the Examples provide adequate directions for preparation of that intermediate.

Reaction Scheme II depicts a novel process for preparation of the novel compounds of formula I.

REACTION SCHEME I

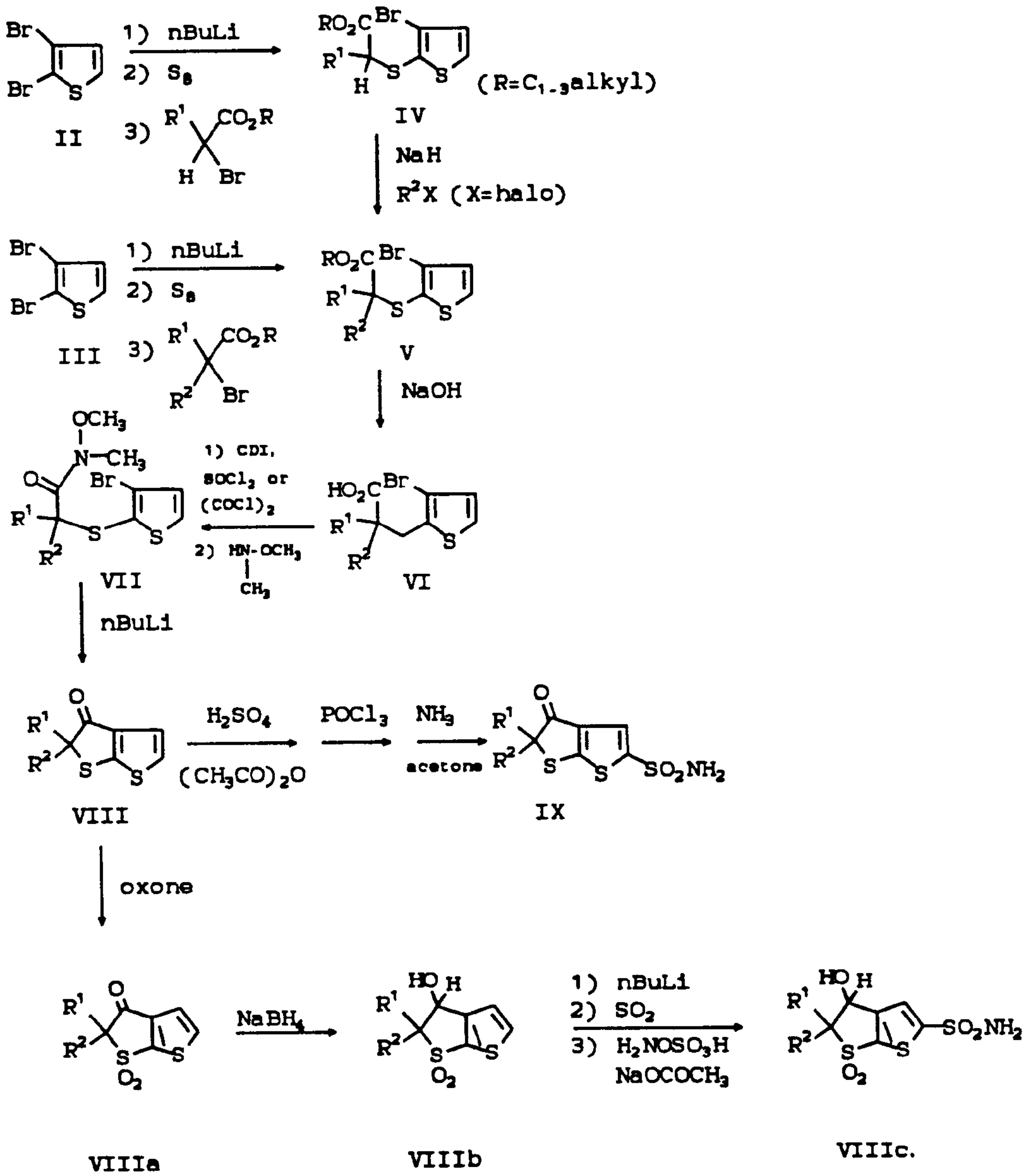


II IV III V VII VI VIII IX VIIIa VIIIb VIIIc.

REACTION SCHEME II

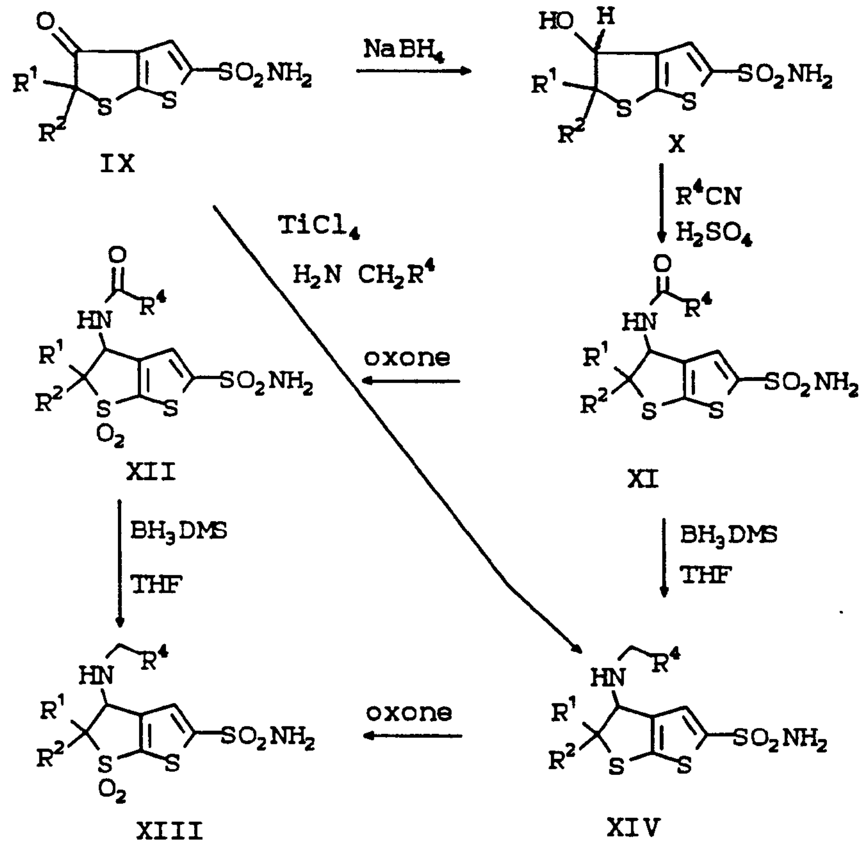


wherein $R^4$ is $C_{1-5}$alkyl

Reduction of the 4-oxo group of compound IX in the reaction scheme to form X is accomplished with a complex metal hydride such as sodium borohydride in a lower alcohol such as ethanol or propanol at about -10 to +10°C for about 1 to 3 hours followed by warming to room temperature. The product may be isolated by addition of water to quench the excess borohydride, evaporation of the alcohol, extraction with ethyl acetate and concentration to dryness.

The amide, XI, is prepared by addition of a nitrile of formula $R^4CN$, cooling to about -15 to +5°C and adding concentrated $H_2SO_4$ dropwise with stirring. After about 0.5 to 2 hours the solution is warmed to room temperature over about 2 to 5 hours. Isolation is accomplished by evaporation of the acetonitrile, dissolving the residue in an aqueous base such as sodium bicarbonate and extracting the product with ethyl acetate.

The sulfone, XII, is prepared by treatment of XI in an absolute lower alcohol such as methanol, ethanol or propanol with oxone or other acceptable oxidizing agent such as $H_2O_2$, for about 12-25 hours at or near room temperature followed by about 10-30 minutes at about 85-100°C. The sulfone is isolated by dilution with water and a weak base such as sodium bicarbonate solution followed by extraction with a solvent such as ethyl acetate.

Reduction of the amide group of XII to the amine of XIII or the amide XI to the amine XIV is accomplished in a solvent such as THF with borane-dimethylsulfide complex at room temperature for about 10 to 30 minutes followed by about 1 to 4 hours at 60-65°C. The product is isolated by cooling to about 0°C quenching the excess $BH_3$ with ethanol and dilute acid, heating for about 15 minutes on steam after evolution of hydrogen ceases, cooling, neutralizing with an aqueous base such as sodium hydroxide or sodium bicarbonate and extraction of the product with a solvent such as ethyl acetate.

The amino compound, XIV, is also prepared by treatment of IX with $TiCl_4$ in a solvent such as THF/benzene at about room temperature followed by addition of an amine of formula $R^4CH_2NH_2$. After about 3 to 7 hours, the mixture is treated with excess sodium bicarbonate solution and extracted with ethyl acetate. The ethyl acetate is evaporated and treated with sodium borohydride in ethanol at about room temperature for about 1 to 5

hours, quenched with aqueous sodium bicarbonate and extracted with ethyl acetate.

The isomers may be separated at compound XI or XII by column chromatography on silica gel by elution with methanol-methylene chloride mixtures.

The novel isomeric structure identified above as II is prepared by processes depicted in Reaction Scheme III.

REACTION SCHEME III

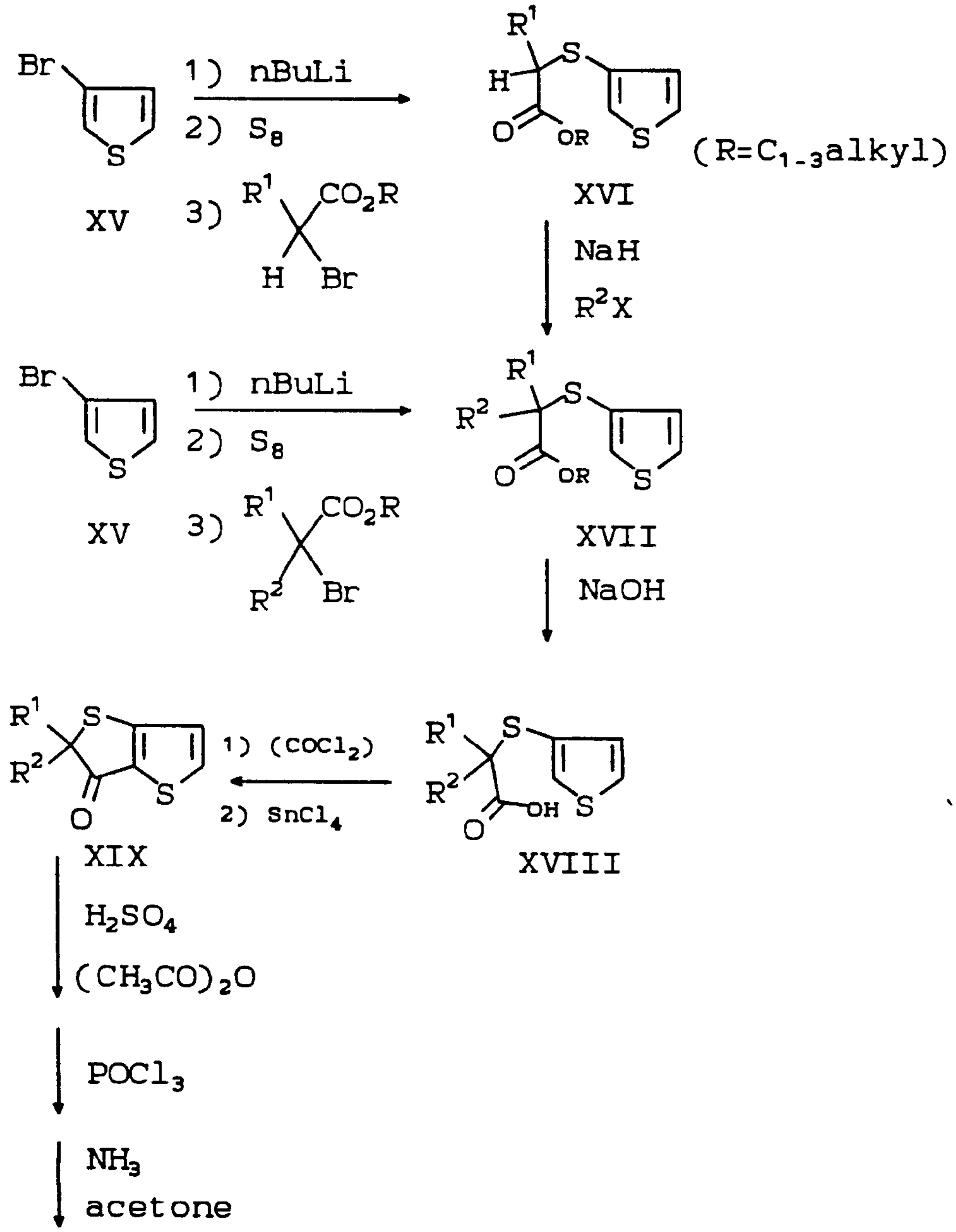

REACTION SCHEME III (CONT'D)

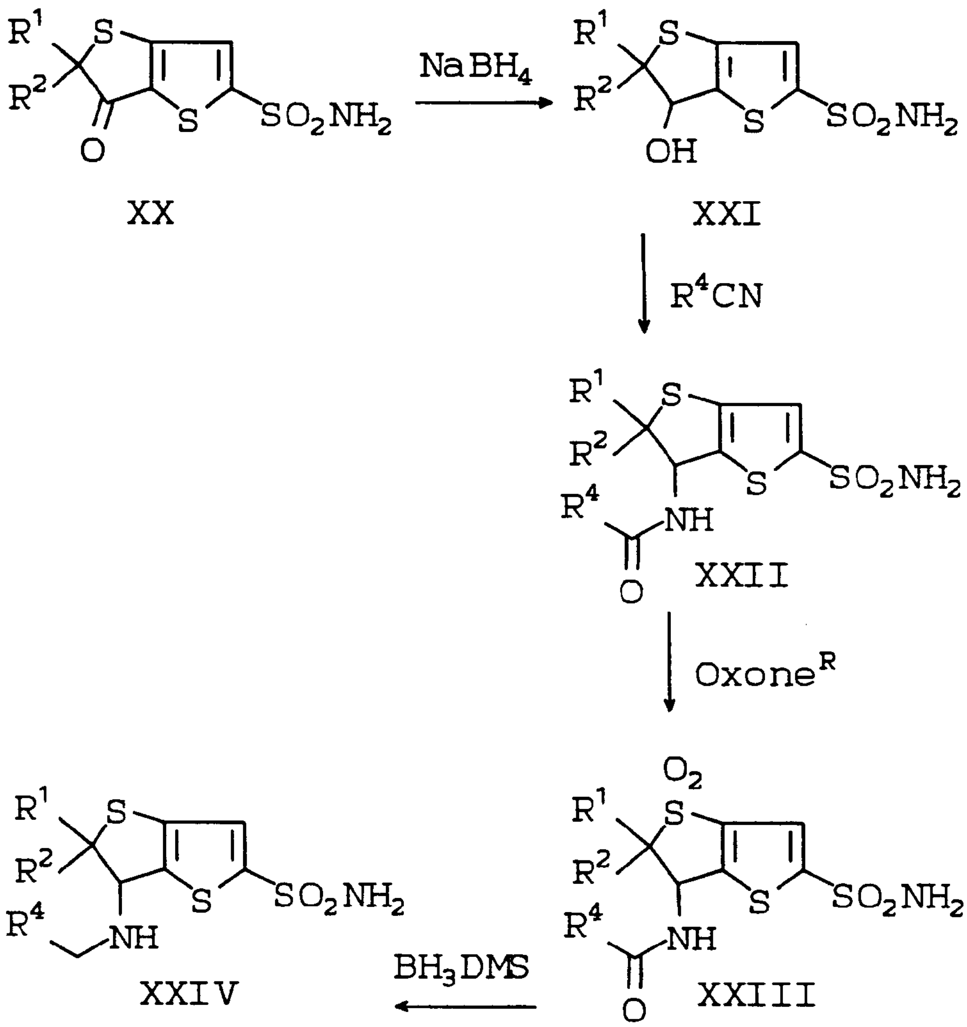


With reference to Reaction Scheme III, a solution in ether at -78°C of 3-bromothiophene XV is treated with nBuLi and then elemental sulfur and then an appropriate 2-bromo ester to give compound XVI. Compound XVI is converted to XVII by treatment with sodium hydride and a suitable alkyl halide in a solvent such as THF or DMF. The ester is hydrolyzed to the acid under standard conditions (NaOH in a lower alcohol) to give compound XVIII. The material is then subjected to standard Friedel-Crafts conditions via the acid chloride to give compound XIX. A sulfonamide group is then introduced by standard means to give XX. Compound XX is treated with sodium borohydride in a lower alcohol to give compound XXI. Treatment of XXI with $R^4CN$ and sulfuric acid gives rise to amide XXII. The sulfide of XX is oxidized to the sulfone oxidation state of XXIII and the amide is reduced to an amine with borane-dimethylsulfide to give the final product XXIV.

Compounds in which $R^2$ is $C_{1-6}$alkyl substituted with an amino or sulfur containing group are prepared in accordance with the processes depicted in Reaction Scheme IV.

REACTION SCHEME IV

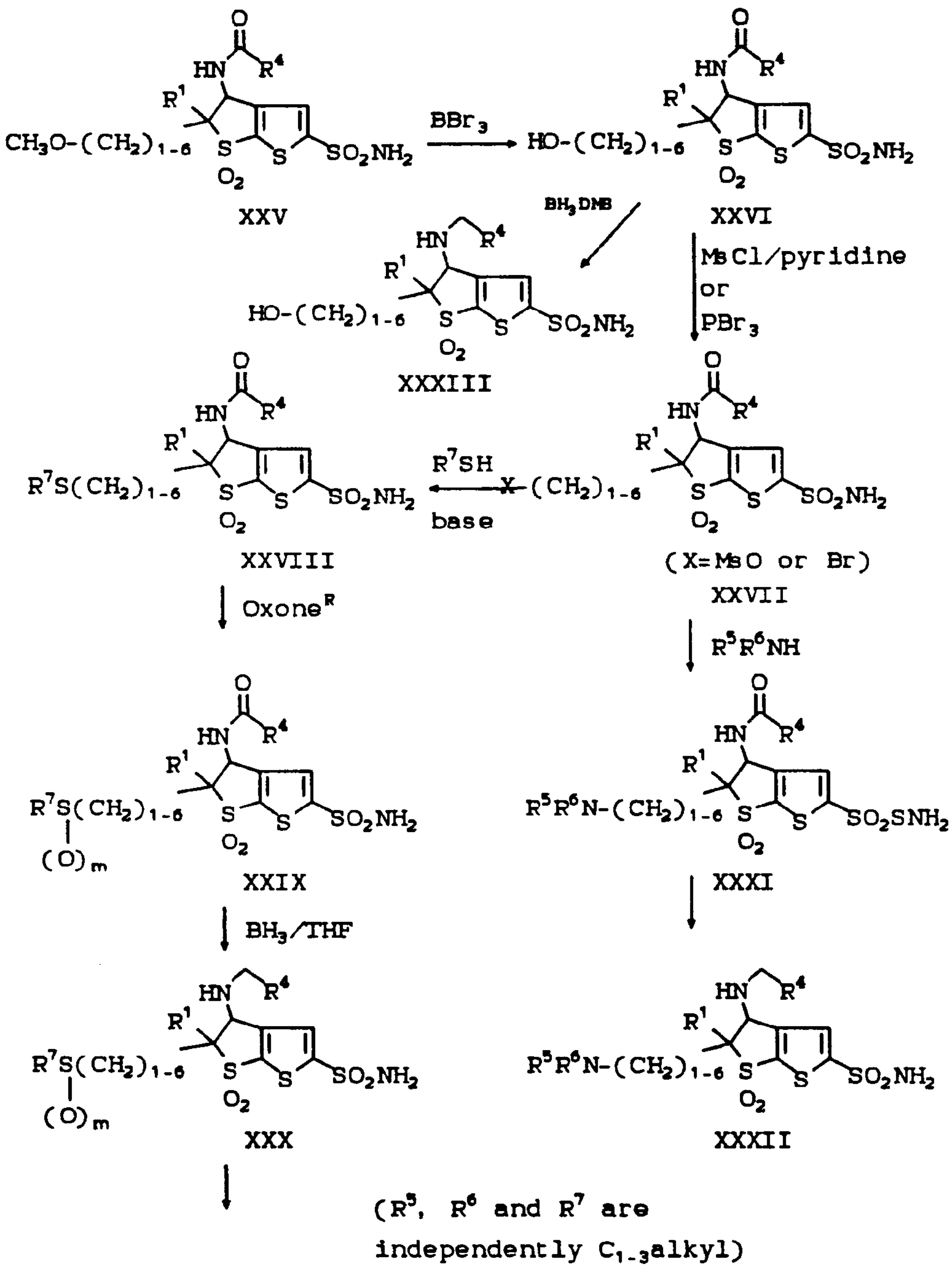


An advanced intermediate such as XXV (XII wherein $R^2 = CH_3O(CH_2)_{1-6}$-) can be demethylated with boron tribromide ($BBr_3$) in methylene chloride to give compound XXVI. The alcohol XXVI can be reduced with borane to the alkylamine XXXIII. The alcohol can then be mesylated with methanesulfonyl chloride or converted to a halide with $PBr_3$. The halide or mesyloxy can be displaced with either a thiolate anion to give XXVIII or an amine nucleophile to produce XXXI. Compound XXVIII can be oxidized to the sulfoxide or sulfone XXIX (n = 1 or 2, respectfully) with Oxone in aqueous alcohol. The final step involves a borane reduction of the amide function in THF to give XXX or XXXII.

## REACTION SCHEME 5

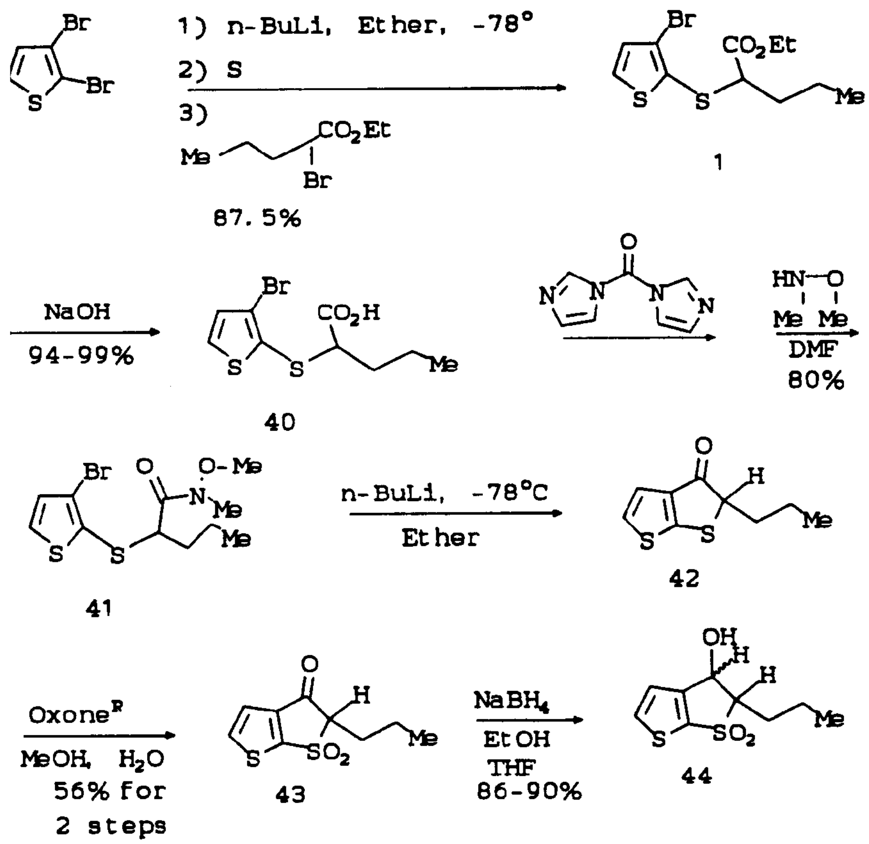


1

40

41

42

43

44

SCHEME 5 Cont'd.

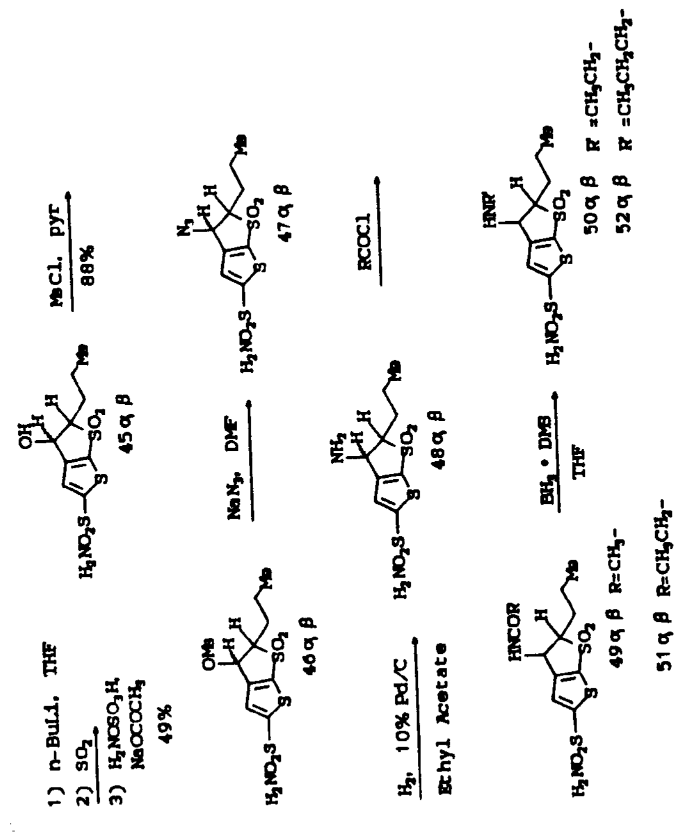

## REACTION SCHEME 6

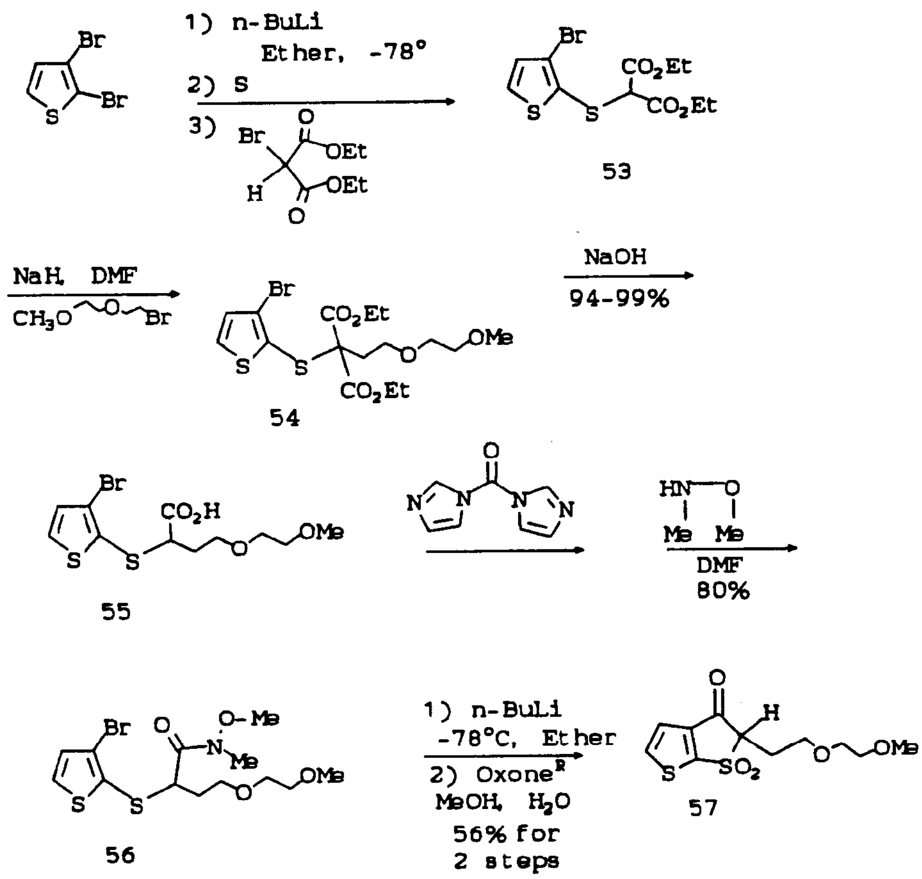


53

54

55

56

57

REACTION SCHEME 6 Cont'd.

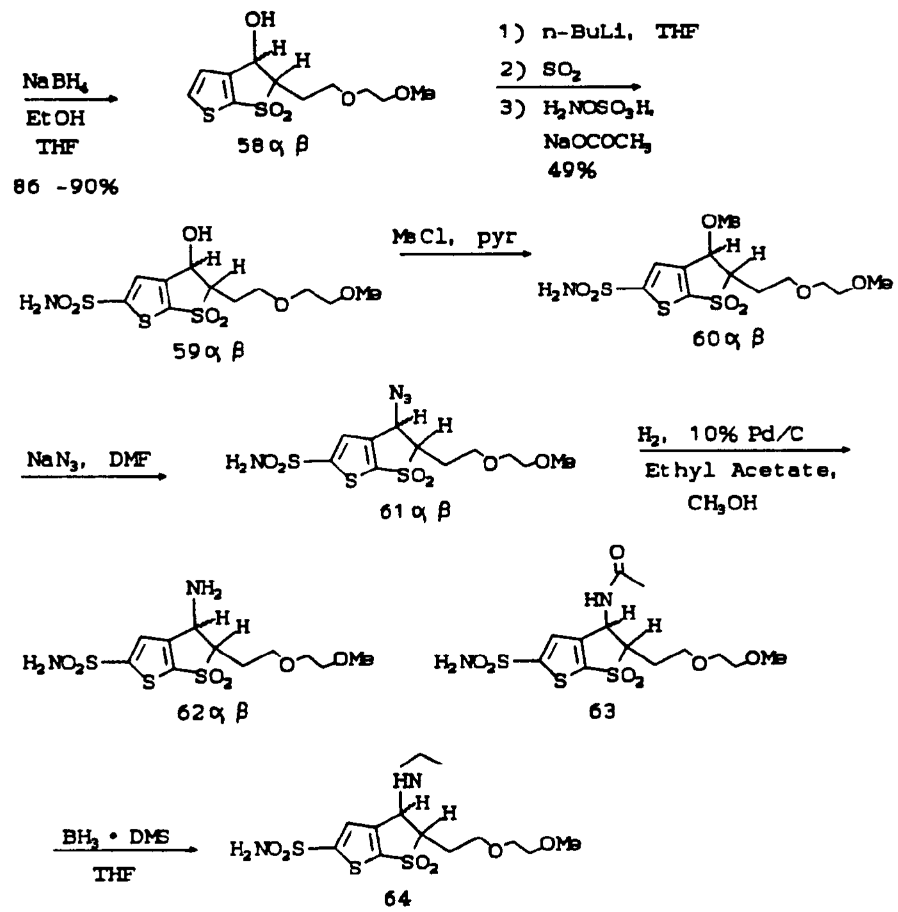


58α β

59α β

60α β

61 α β

62α β

63

64

## REACTION SCHEME 7

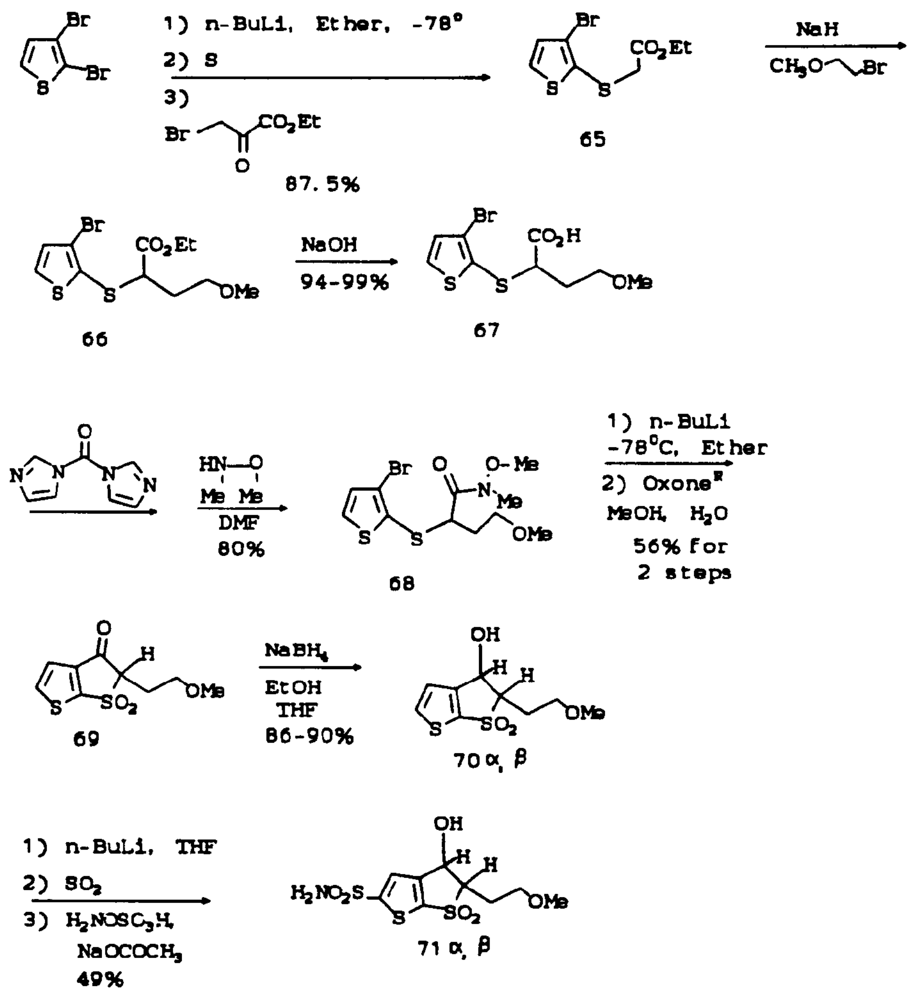


The novel pharmaceutical formulations of this invention can be adapted for oral administration such as tablets, capsules or the like; for nasal administration, especially in the form of a spray; for injection, in the form of a sterile injectable liquid; or for topical ocular administration in the form of solutions, suspensions, ointments, solid water soluble polymeric inserts, or gels.

This invention is particularly concerned with formulations adapted for topical ocular administration for the treatment of glaucoma and other stages of elevated intraocular pressure and contain about 0.1% to 15% by weight of medicament, especially about 0.5 to 2% by weight of medicament, the remainder being comprised of carriers and other excipients well known in the art.

The medicament in the novel topical ocular formulations comprises one of the novel compounds of this inventions either alone or in combination with a β-adrenergic blocking agent such as timolol maleate or a parasympathomimetic agent such as pilocarpine or other antiglaucoma agents which include forskolin derivatives, $\alpha_2$ agonists and antagonists, renin inhibitors, ACE inhibitors, Angiotensin II inhibitors, prostaglandins, atrial natriuretic peptides, $D_2$-agonists, phosphodiesterase inhibitors, $K^+$ channel openers, $Ca^{++}$ channel blockers, cannabinoido and endothelin.

In such combinations the two active agents are present in approximately pharmacologically equal amounts.

The novel method of treatment of this invention comprises the treatment of elevated intraocular pressure by the administration of a novel compound of this invention or a pharmaceutical formulation thereof. Of primary concern is the treatment by topical ocular administration of about 0.1 to 25 mg and especially 0.2 to 10 mg of

such compound per day, either by single dose or on a 2 to 4 dose per day regimen.

The following examples illustrate the present invention, without, however limiting the same thereto.

Example 1

α and β Isomers of 4,5-Dihydro-4-ethylamino-5-methyl-5-propyl-4H-thieno[2,3-b]thiophene-2-sulfonamide-6,6-dioxide

Step A: Preparation of Ethyl 2-(3-bromo-2-thienylthio)-valerate:

To a solution of 2,3-dibromothiophene (50.0 g, 0.207 mol) in diethyl ether (500 ml) at -78°C was added, over 30 minutes, n-butyllithium (95.0 ml, 0.238 mol) as a 2.5 M solution in hexanes. The reaction was allowed to stir an additional 1 hour. To this solution was added powdered sulfur (8.80 g, 0.274 mol) and the reaction was allowed to stir at -78°C for 15 minutes, warmed to -30°C over 45 minutes, and then cooled again to -78°C. To this mixture was added ethyl 2-bromovalerate (60.7 g, 0.335 mol) and the resulting mixture was allowed to warm to room temperature for 16 hours. The reaction was quenched with water and aqueous sat. $NaHCO_3$ and the aqueous was extracted with ethyl acetate (3 x 300 ml). The combined extracts were washed with sat. brine (250 ml) and dried over $MgSO_4$. The solvent was removed by evaporation in vacuo. The product was purified twice by flash chromatography with silica gel and 1-4% ethyl acetate/hexane as eluant to yield 58.54 g (87.5%) of product as a yellow oil:
$^1H$ NMR (δ from TMS in $CDCl_3$ in ppm): 7.38 (d, 1H, J=5.4 Hz), 7.03 (d, 1H, J=5.4 Hz), 4.13 (q, 2H, J=7.1 Hz), 3.58 (dd, 1H, J=8.1 Hz), 1.89-1.75 (m, 2H), 1.53-1.43 (m, 2H), 1.21 (t, 3H, J=7.1 Hz), 0.95 (t, 3H, J=7.3 Hz).

Step B: Preparation of Ethyl 2-(3-bromo-2-thienylthio)-2-methyl-valerate:

To a mixture of sodium hydride (6.77 g, 0.169 mol, NaH) as a 60% dispersion in mineral oil in tetrahydrofuran (300 ml), at 0°C, was added iodomethane (25.0 ml, 57.0 g, 0.402 mol) followed by the product from step A (44.83 g, 0.1387 mol) in tetrahydrofuran (100 ml). The reagents were added carefully to avoid rapid hydrogen gas evolution or an exothermic reaction. The mixture was then allowed to warm to room temperature and stir overnight, about 19 hours. Proton NMR showed the reaction was incomplete; therefore, iodomethane (10.0 ml, 22.8 g, 0.161 mol) was added and the reaction allowed to stir for 20 hours. To the solution was added additional sodium hydride (3.00 g, 0.075 mol) as a 60% dispersion in mineral oil and iodomethane (10.0 ml, 22.8 g, 0.161 mol) and the mixture allowed to stir at room temperature for 16 hours. The reaction mixture was quenched with aqueous sat. $NaHCO_3$, diluted with diethyl ether, and the layers were separated. The aqueous was extracted with diethyl ether (4 x 150 mol) and the combined ethereal extracts were washed with 1 N NaOH (4 x 50 ml) and dried over $MgSO_4$. The product was purified by flash chromatography to yield 37.0 g (79%) of product as a yellow oil:
$^1H$ NMR (δ from TMS in $CDCl_3$ in ppm): 7.44 (d, 1H, J=5.5 Hz), 7.06 (d, 1H, J=5.5 Hz), 4.22-4.13 (m, 2H), 1.96-1.90 (m, 1H), 1.78-1.68 (m, 1H), 1.53-1.44 (m, 1H), 1.46 (s, 3H), 1.33-1.24 (m, 1H), 1.26 (t, 3H, J=7.1 Hz), 0.94 (t, 3H, J=7.2 Hz).

Step C: Preparation of 2-(3-bromo-2-thienylthio)-2-methyl-valeric acid:

To a solution of the product from step B (7.30 g, 21.6 mmol) in methanol (100 ml) was added 10 N sodium hydroxide solution (120 ml) and the resulting mixture was heated to reflux for 5 hours then simmered overnight, about 16 hours. The reaction mixture was cooled to room temperature and extracted with diethyl ether (2 x 100 ml). The ether layer product was washed with 1 N NaOH (4 x 100 ml). The alkaline washes were made acidic with conc. HCl and extracted with diethyl ether (4 x 100 ml). The original aqueous was made acidic and extracted with ether (4 x 100 ml). The combined organic extracts from acid were dried over $MgSO_4$ and concentrated to yield 6.42 g (96%) of the acid product as an orange oil:
$^1H$ NMR (δ from TMS in $CDCl_3$ in ppm): 11.05 (vb, 1H), 7.45 (d, 1H, J=5.5 Hz), 7.07 (d, 1H, J=5.5 Hz), 1.96-1.90 (m, 1H), 1.79-1.68 (m, 1H), 1.57-1.50 (m, 1H), 1.46 (s, 3H), 1.41-1.33 (m, 1H), 0.96 (t, 3H, J=7.2 Hz).

Step D: Preparation of 2-(3-bromo-2-thienylthio)-2-methyl-N-methoxy-valeramide:

To a solution of the product from step C (3.89 g, 12.6 mmol) in methylene chloride (130 ml) at 0°C was added N,N′-dimethyl formamide (0.35 ml, 4.5 mmol) followed by oxalyl chloride (1.59 ml, 2.18 g, 17.2 mmol). The ice bath was removed and the reaction allowed to stir for 1.5 hours or until the evolution of gas ceased.

The solvent and excess oxalyl chloride were removed by evaporation in vacuo to yield a gummy solid. To this was added 30 ml pyridine and a mixture of N,O-dimethylhydroxylamine hydrochloride (3.90 g, 37.6 mmol), 1-methylpiperidine (4.60 ml, 3.75 g, 37.8 mmol) and pyridine (70 ml). The resulting mixture was allowed to stir at room temperature for 4.5 hours. The pyridine was removed by evaporation in vacuo, and the residue was dissolved in ethylacetate (100 ml) and washed with 1 N HCl (4 x 50 ml) and with 1 N NaOH (4 x 50 ml). The ethyl acetate was dried over $MgSO_4$ and concentrated. The product was purified with 8-12% ethyl acetate-/hexane as eluant to yield 3.93 g (88.6%) of the amide product as a pale yellow oil:
$^1H$ NMR (δ from TMS in $CDCl_3$ in ppm): 7.40 (d, 1H, J=5.5 Hz), 7.04 (d, 1H, J=5.5 Hz), 3.86 (s, 3H), 3.27 (s, 3H), 2.22-2.12 (m, 1H), 1.76-1.66 (m, 1H), 1.49-1.41 (m, 1H), 1.42 (s, 3H), 1.29-1.23 (m, 1H), 0.93 (t, 3H, J=7.1 Hz).

Step E: Preparation of 4,5-Dihydro-5-methyl-5-propyl-4-oxo-4H-thieno[2,3-b]thiophene:

To a solution of the amide product from step D (10.28 g, 0.0292 mol) in anhydrous diethyl ether (200 ml) at -78°C was added, over 20 minutes, n-butyllithium (16.0 ml, 40.0 mmol) as a ~ 2.5 M solution in hexanes and the solution was allowed to stir for an additional 40 minutes at -78°C. Care was taken to keep the temperature at -78°C during addition. To the solution was added aqueous sat. $NaHCO_3$ (60 ml) and water (50 ml) and the mixture was allowed to warm to room temperature. The layers were separated and the aqueous was extracted with diethyl ether (2 x 50 ml) and the combined organic extracts were washed with sat. brine (100 ml) and dried over $MgSO_4/NaHCO_3$ and concentrated. The product was purified by flash chromatography on silica gel and eluted with 3% ethyl acetate/hexane to yield 5.90 g (95%) of the ketone product as a yellow oil:
$^1H$ NMR (δ from TMS in $CDCl_3$ in ppm): 7.17 (dd, 2H, J=5.3 Hz), 1.96-1.76 (m, 2H), 1.62 (s, 3H), 1.55-1.46 (m, 1H), 1.28-1.22 (m, 1H), 0.90 (t, 3H, J=7.4 Hz); $^{13}C$ NMR (δ in $CDCl_3$ in ppm): 196.64, 166.26, 137.52, 128.30, 121.58, 74.02, 41.71, 25.41, 18.59, 14.08.

Step F: Preparation of 4,5-Dihydro-5-methyl-5-propyl-4-oxo-4H-thieno[2,3-b]thiophene-2-sulfonamide

To a solution of the product from step E (8.172 g, 38.49 mmol) in methylene chloride (40 ml) was added acetic anhydride (11.0 ml, 11.9 g, 116.6 mmol) and, at 0°C, concentrated sulfuric acid (2.4 ml, 43.2 mmol). The solution was stirred with warming to room temperature over 5 hours. The solvent was removed by evaporation in vacuo and the residue was dissolved in acetonitrile (38 ml) and tetramethylenesulfone (38 ml). To this was added N,N'-dimethylacetamide (1.60 ml, 1.50 g, 17.2 mmol), triethylamine (6.50 ml, 4.72 g, 46.6 mmol) and, at 0°C, phosphorus oxychloride (8.40 ml, 13.8 g, 90.1 mmol). The resulting solution was warmed to room temperature and allowed to stir about 24 hours. The solution was poured into ice and extracted with ethyl acetate (3 x 100 ml). The combined extracts were concentrated and dissolved in acetone (75 ml). To this solution was added, at 0°C, dropwise with stirring, concentrated ammonium hydroxide (50 ml). The mixture was allowed to warm to room temperature and stir for 4 hours. The solvent and ammonia were removed by evaporation in vacuo and the product mixture was poured into ice-water and extracted with ethyl acetate (3 x 150 ml). The combined extracts were dried over $MgSO_4$ and concentrated. The product was purified by flash chromatogrphy on silica gel and 20-25% ethyl acetate/hexane as eluant to yield an off-white solid. The product was crystallized from diethyl ether/hexane to yield 6.739 g (60%) of sulfonamide product as white, floculent crystals: mp = 93-95°C;
$^1H$ NMR (δ from TMS in DMSO-$d_6$ in ppm): 7.83 (s, 2H), 7.48 (s, 1H), 3.36 (bs, 1H), 1.85-1.79 (m, 2H), 1.54 (s, 3H), 1.44-1.35 (m, 1H), 1.15-1.06 (m, 1H), 0.84 (t, 3H, J=7.1 Hz);

| | |
|---|---|
| Anal. Calcd. for $C_{10}H_{13}NS_3O_3$: | C, 41.22; H, 4.50; N, 4.81. |
| Found: | C, 41.27; H, 4.31; N, 4.76. |

Step G: Preparation of 4,5-Dihydro-4-acetomido-5-methyl-5-propyl-4H-thieno[2,3-b]thiophene-2-sulfonamide

A solution of the product from step F (1.32 g, 4.53 mmol) in dry tetrahydrofuran (15 ml) was added to a stirring mixture of sodium borohydride (0.20 g, 5.3 mmol) as a 60% dispersion in mineral oil in absolute ethanol (15 ml) at 0°C and the resulting mixture was stirred over 2 hours with warming to room temperature. The excess borohydride was quenched by addition of water followed by addition of 1 N HCl and the tetrahydrofuran was then removed by evaporation in vacuo. The aqueous was extracted with ethyl acetate (4 x 30 ml) and the combined extracts were dried over $MgSO_4$. The solvent was removed by evaporation in vacuo and to the residue was added acetonitrile (100 ml). The resulting solution was cooled to -10 -0°C and to this was added, dropwise over 15 minutes, concentrated sulfuric acid (0.50 ml) and the reaction mixture was allowed to stir for 1 hour at 0°C and then warmed to room temperature for 3 hours. The acetonitrile was removed by evaporation in vacuo, and the residue was dissolved in water and aq. sat. $NaHCO_3$ and extracted with ethyl acetate ( 3 x 50 ml). The combined extracts were dried over $MgSO_4$ and concentrated. The product was purified by flash chromatography on silica gel and 30 - 50% ethyl acetate/hexane as eluant to yield 0.95 g (63%) of the acetamido product as a white solid, as a mixture of inseparable isomers: mp = 170-174°C;
$^1$H NMR (δ from TMS in DMSO-$d_6$ in ppm): 8.41 (d, 1H, J=8.6 Hz), 8.39 (d, 1H, J=8.6 Hz), 7.59 (s, 4H), 7.27 (s, 1H), 7.24 (s, 1H), 5.18 (d, 1H, J=9.0 Hz), 5.07 (d, 1H, J=9.0 Hz), 1.89 (s, 3H), 1.87 (s, 3H), 1.81-1.62 (m, 4H), 1.53 (s, 3H), 1.38 (s, 3H), 1.53-1.38 (m, 2H), 1.26-1.18 (m, 2H), 0.92-0.86 (m, 6H).

Step H: Preparation of 4,5-Dihydro-4-acetamido-5-methyl-5-propyl-4H-thieno[2,3-b]thiophene-2-sulfonamide-6,6-dioxide:

To a solution of the product of step G (0.95 g, 2.84 mmol) in absolute ethanol (30 ml) and water (10 ml) was added oxone (3.50 g, 5.69 mmol) as a solution in water (40 ml) and the resulting mixture was stirred at room temperature for 15 hours. The mixture was warmed on a steam bath for 20 minutes and then cooled to room temperature and diluted with water and sodium bicarbonate. The aqueous was extracted with ethyl acetate (3 x 75 ml) and the combined extracts were dried over $MgSO_4$. This product was combined with another run of an identical reaction. Purified by flash chromatography with silica gel and 2-3% methanol/methylene chloride to give 0.732 g (49%) of α-isomer, mp = 174-176°C;
$^1$H NMR (δ from TMS in DMSO-$d_6$ in ppm): 8.56 (d, 1H, J=8.8 Hz), 8.09 (s, 2H), 7.53 (s, 1H), 5.31 (d, 1H, J=8.8 Hz), 1.93-1.82 (m, 1H), 1.92 (s, 3H), 1.64-1.58 (m, 1H), 1.45 (s, 3H), 1.45-1.36 (m, 2H), 0.91 (t, 3H, J=7.1 Hz); and 0.557 g (37%) of the β-isomer: mp = 118°C with decomposition;
$^1$H NMR (δ from TMS in DMSO-$d_6$ in ppm): 8.64 (d, 1H, J=9.1 Hz), 8.10 (s, 2H), 7.53 (s, 1H), 5.42 (d, 1H, J=9.1 Hz), 1.96 (s, 3H), 2.00-1.89 (m, 1H), 1.80-1.70 (m, 1H), 1.47-1.28 (m, 2H), 1.30 (s, 3H), 0.91 (t, 3H, J=7.1 Hz).

Step I: Preparation of 4,5-Dihydro-4-ethylamino-5-methyl-5-propyl-4H-thieno[2,3-b]thiophene-2-sulfonamide-6,6-dioxide α-isomer:

To a solution of the product of step H (0.64 g, 1.75 mmol) in tetrahydrofuran (25 ml) was added $BH_3$ (0.50 ml, 5.0 mmol) as a 10 M borane-dimethylsulfide complex solution. The resulting solution was stirred at room temperature for 20 minutes and heated to 60-64°C for 1 hour. More $BH_3$ (0.18 ml, 1.8 mmol) was added and the solution heated for 1 hour. The reaction mixture was cooled to 0°C and quenched with ethanol followed by water and 1 N HCl. When $H_2$ gas evolution ceased, the reaction mixture was heated on a steam bath for 15 minutes, cooled to room temperature, and made neutral with 10 N NaOH and $NaHCO_3$. The cloudy aqueous solution was extracted with ethyl acetate (3 x 75 ml) and the combined extracts were dried over $MgSO_4$ and concentrated. The product was purified by flash chromatography on silica gel and 1% methanol/methylene chloride as eluant to yield 0.528 g (86%) of 10α as an oil. The product was dissolved in absolute ethanol and 1 eq. of HCl as a 3.22 M ethanolic HCl solution was added to the solution. The product was allowed to crystallize from ethanol/diethyl ether to yield 0.437 g (57%) of α-isomer: mp = 212-216.5°C with decomposition; $^1$H NMR (δ from TMS in DMSO-$d_6$ in ppm): 10.18 (b, 1H), 9.25 (b, 1H), 8.21 (bs, 2H), 7.93 (s, 1H), 4.88 (bs, 1H), 3.17-2.92 (bm, 2H) 2.28 (bm, 1H), 2.03 (bm, 1H), 1.16-1.46 (m, 2H), 1.51 (s, 3H), 1.28 (t, 3H, J=7.1 Hz), 0.98 (t, 3H, J=7.2 Hz);

Anal. Calcd. for $C_{12}H_{20}N_2S_3O_4$•HCl•$C_2H_6O$:
C, 38.65; H, 6.03; N, 6.44.
Found: C, 38.34; H, 6.02; N, 6.62.

Step J: Preparation of 4,5-Dihydro-4-ethylamino-5-methyl-5-propyl-4H-thieno[2.3-b]thiophene-2-sulfonamide-6,6-dioxide β-isomer:

To a solution of the β-isomer from step H amide sulfone 9β (0.48 g, 1.3 mmol) in tetrahydrofuran (20 ml) was added, dropwise over 5 minutes, of $BH_3$ (0.40 ml, 4.0 mmol) as a 10 M borane-dimethylsulfide complex solution and the resulting solution was stirred at room temperature for 20 minutes and heated to 60-64°C for

1 hour. More borane (0.12 ml, 1.2 mmol) was added and the solution heated for 1 hour. The reaction mixture was cooled to room temperature and quenched with ethanol followed by water and 1 N HCl. When no $H_2$ evolution could be observed, the reaction mixture was heated on a steam bath for 15 minutes, cooled to room temperature and made neutral with 10 N NaOH and $NaHCO_3$. The cloudy aqueous solution was extracted with ethyl acetate (3 x 75 ml). The combined extracts were dried over $MgSO_4$ and concentrated. The product was purified by flash chromatography with silica gel and 1% methanol/methylene chloride as eluant to yield 0.361 g (78%) of 10β as the free base. The product was dissolved in absolute ethanol and to this was added 1 eq. HCl as a 3.22 M ethanolic HCl solution. The product was allowed to crystallize from ethanol/diethyl ether to yield 0.177 g (35%) of the product as the HCl salt: mp = 228-232°C with decomposition;
$^1H$ NMR (δ from TMS in DMSO-$d_6$ in ppm): 10.21 (bs, 1H), 9.73 (bs, 1H), 8.22 (s, 2H), 7.93 (s, 1H), 4.98 (bm, 1H), 3.64 (bm, 2H), 2.05-1.95 (bm, 2H), 1.65 (bs, 3H), 1.59-1.24 (m, 5H), 0.91 (t, 3H, J=7.0 Hz);

Anal. Calcd. for $C_{12}H_{20}N_2S_3O_4 \bullet HCl$:
C, 37.05; H, 5.18; N, 7.20.
Found: C, 37.39; H, 5.45; N, 7.06.

Example 2

α and β-Isomer of 4,5-Dihydro-5-methyl-5-propyl-4-propylamino-4H-thieno[2,3-b]thiophene-2-sulfonamide-6,6-dioxide.

Step A: Preparation of 4,5-Dihydro-5-methyl-5-propyl-4-propionamido-4H-thieno[2,3-b]thiophene-2-sulphonamide:

To a mixture of sodium borohydride (0.47 g, 12.4 mmol) in absolute ethanol (20 ml) and tetrahydrofuran (50 ml) at 0°C was added the ketone product from step F of example 1 (2.144 g, 7.36 mmol) in tetrahydrofuran (12 ml) and the resulting mixture was stirred with warming to room temperature for 2 hours. The reaction was quenched with water and 1 N HCl and the aqueous was made basic with sat. sodium bicarbonate solution. The aqueous was extracted with ethyl acetate (4 x 75 ml) and the combined extracts were dried over $MgSO_4$ and concentrated. The resulting white foam was dissolved in propionitrile (40 ml) and cooled to -40°C. Concentrated $H_2SO_4$ (0.40 ml) in propionitrile (8 ml) was added dropwise to the solution. After 30 minutes, the reaction mixture was warmed to 0°C, concentrated $H_2SO_4$ (0.30 ml) was added and the reaction was allowed to stir for 5 hours. The reaction mixture was diluted with water and quenched with sodium bicarbonate. The aqueous was extracted with ethyl acetate (2 x 200 ml) and the combined extracts were dried over $MgSO_4$ and concentrated. The product was purified by flash chromatography on silica gel and 35-50% ethyl acetate/hexane as eluant to yield 1.300 g (51%) of the amide as a mixture of inseparable isomers: mp = 153-165°C. major isomer:
$^1H$ NMR (δ from TMS in DMSO-$d_6$ in ppm): 8.31 (d, 1H, J=9.2 Hz), 7.59 (s, 2H), 7.26 (s, 1H), 5.09 (d, 1H, J=9.2 Hz), 2.21-2.10 (m, 2H), 1.80-1.76 (m, 1H), 1.74-1.63 (m, 1H), 1.53 (s, 3H), 1.54-1.36 (m, 1H), 1.24-1.18 (m, 1H), 1.02 (m, 3H), 0.89 (t, 3H, J=7.1 Hz). minor isomer:
$^1H$ NMR (δ from TMS in DMSO-$d_6$ in ppm): 8.33 (d, 1H, J=8.7 Hz), 7.59 (s, 2H), 7.23 (s, 1H), 5.21 (d, 1H, J=8.7 Hz), 2.21-2.10 (m, 2H), 1.80-1.76 (m, 1H), 1.74-1.63 (m, 1H), 1.38 (s, 3H), 1.54-1.36 (m, 1H), 1.24-1.18 (m, 1H), 1.02 (m, 3H), 0.89 (t, 3H, J=7.1 Hz).

Step B: Preparation of 4,5-Dihydro-5-methyl-5-propyl-4-propionamido-4H-thieno[2,3-b]thiophene-2-sulfonamide-6,6-dioxide:

To a solution of product from step A (1.289 g, 3.70 mmol) in methanol (40 ml) was added half of a solution of oxone (4.59 g, 7.47 mmol) in water (50 ml). Upon addition of the oxone to the methanol, the oxone fell out of solution. The reaction mixture was cooled to 0°C and the remaining oxone solution was added. The reaction mixture was allowed to warm to room temperature while stirring overnight, ca 21 hours. The reaction was quenched with aqueous sat. $NaHCO_3$ and extracted with ethyl acetate (4 x 75 ml). The combined extracts were dried over $MgSO_4$ and concentrated. This was combined with two previous reactions carried out in an identical fashion. The product was purified by flash chromatography on silica gel and eluted with 1-2.5% methanol/methylene chloride to yield 0.985 (38%) of α-isomer:
$^1H$ NMR (δ from TMS in DMSO-$d_6$ in ppm): 8.53 (d, 1H, J =8.8 Hz), 8.10 (s, 2H), 7.53 (s, 1H), 5.33 (d, 1H, J=8.8

Hz), 2.24-1.95 (m, 2H), 1.94-1.84 (m, 1H), 1.64-1.56 (m, 1H), 1.47-1.36 (m, 2H), 1.46 (s, 3H), 1.03 (t, 3H, J=7.5 Hz), 0.90 (t, 3H, J=7.1 Hz) and β-isomer:
$^1H$ NMR (δ from TMS in DMSO-$d_6$ in ppm): 8.58 (d, 1H, J=9.0 Hz), 8.09 (s, 2H), 7.51 (s, 1H), 5.43 (d, 1H, J=9.0 Hz), 2.27-2.20 (m, 2H), 1.98-1.90 (m, 1H), 1.88-1.70 (m, 1H), 1.47-1.32 (m, 2H), 1.29 (s, 3H), 1.06 (t, 3H, J=7.1 Hz), 0.91 (t, 3H, J=7.1 Hz),

Step C: Preparation of 4,5-Dihydro-5-methyl-5-propyl-4-propylamino-4H-thieno[2,3-b]thiophene-2-sulfonamide-6,6-dioxide α-isomer:

To a solution of α-isomer product from step B (0.819 g, 2.15 mmol) in tetrahydrofuran (30 ml) was added $BH_3$ (0.54 ml, 5.4 mmol) as a 10 M boranedimethylsulfide complex solution. The resulting solution was stirred at room temperature for 20 minutes and heated to 60-64°C for 2 hours. The addition of more $BH_3$ (0.30 ml, 3.0 mmol) to the solution was followed by heating for 0.5 hours. The reaction mixture was cooled to 0°C and quenched with ethanol followed by water and 1 N HCl. When $H_2$ gas evolution ceased, the reaction mixture was heated on a steam bath for 15 minutes, cooled to room temperature, and made neutral with 10 N NaOH and $NaHCO_3$. The aqueous solution was extracted with ethyl acetate (3 x 100 ml). The combined extracts were dried over $MgSO_4$ and concentrated. The product was purified by flash chromatography with silica gel and 1% methanol/methylene chloride as eluant to yield 0.586 g (74.4%) of 13α as an oil. The product was dissolved in absolute ethanol and 1 eq. of HCl as a 3.22 M ethanolic HCl solution was added to the solution. The product was allowed to crystallize from ethanol/diethyl ether to yield 0.444 g (50%) of the title compound mp = 243-245°C with decomposition;
$^1H$ NMR (δ from TMS in DMSO-$d_6$ in ppm): 10.28 (bs, 1H), 9.35 (bs, 1H), 8.22 (s, 2H), 7.96 (s, 1H), 4.87 (bm, 1H), 3.84 (bm, 2H), 2.90-2.82 (bm, 1H), 2.77-2.69 (bm, 1H), 2.33-2.21 (bm, 1H), 2.05-1.81 (m, 1H), 1.76-1.35 (m, 2H), 1.51 (s, 3H), 0.98 (t, 3H, J=7.1 Hz), 0.90 (t, 3H, J=7.3 Hz); Anal. Calcd. for $C_{13}H_{22}N_2S_3O_4 \cdot HCl \cdot 0.15$ $C_2H_6O$:

C, 38.97; H, 5.88; N, 6.84.
Found: C, 39.03; H, 5.89; N, 6.80.

Step D: Preparation of 4,5-Dihydro-5-methyl-5-propyl-4-propylamino-4H-thieno[2,3-b]thiophene-2-sulfonamide-6,6-dioxide β-isomer:

To a solution of the β-isomer product from step B (0.559 g, 1.47 mmol) in tetrahydrofuran (20 ml) was added $BH_3$ (0.36 ml, 3.6 mmol) as a 10 M borane-dimethylsulfide complex solution. The resulting solution was stirred at room temperature for 20 minutes and heated to 60-64°C for 2 hours. The addition of more $BH_3$ (0.20 ml, 2.0 mmol) to the solution was followed by heating for 0.5 hours. The reaction was cooled to 0°C and quenched with ethanol followed by water and 1 N HCl. When $H_2$ gas evolution ceased, the reaction mixture was heated on a steam bath for 15 minutes, cooled to room temperature, and made neutral with 10 N NaOH and $NaHCO_3$. The aqueous solution was extracted with ethyl acetate (3 x 50 ml) and the combined extracts were dried over $MgSO_4$ and concentrated. The product was purified by flash chromatography with silica gel and 1% methanol/ methylene chloride as eluant to yield 0.433 g (80.4%) of 13β as an oil. The product was dissolved in absolute ethanol and 1 eq. of HCl as a 3.22 M ethanolic HCl solution was added to the solution. The product was allowed to crystallize from ethanol/diethyl ether to yield 0.307 g (46.5%) of title compound: mp=169-172°C with decomposition;
$^1H$ NMR (δ from TMS in DMSO-$d_6$ in ppm): 10.31 (bs, 1H), 9.82 (bs, 1H), 8.23 (s, 2H), 7.97 (s, 1H), 4.97 (bm, 1H), 3.81 (bm, 2H), 2.08-1.98 (bm, 2H), 1.85-1.71 (m, 2H), 1.65 (s, 3H), 1.46-1.36 (m, 2H), 1.06-0.88 (overlapping t, 6H, J=7.1 Hz, J=7.4 Hz);

Anal. Calcd. for $C_{13}H_{22}N_2S_3O_4 \bullet HCl \bullet C_2H_6O$:
C, 40.12; H, 6.51; N, 6.24.
Found: C, 39.98; H, 6.33; N, 6.15.

Example 3

α and β-Isomers of 4,5-Dihydro-4-ethylamino-4H-5-(2-methoxyethyl)-5-methylthieno[2,3-b]thiophene-2-sulfonamide-6,6-dioxide

Step A: Preparation of ethyl 2-bromo-(3-bromo-2-thienylthio)propionate

The title compound was prepared in 64% yield from 2,3-dibrmothiophene (60 g, 247 mmol) and ethyl 2-bromopropionate (45.3-g, 250 mmol) in the manner described for the preparation of the product of Example 1, Step A.
$^1H$ NMR ($CDCl_3$), δ: 7.40 (d, J=5.6 Hz, 1H), 7.05 (d, J=5.6 Hz, 1H), 4.14 (q, J=7.0 Hz, 2H), 3.73 (q, J=7.2 Hz, 1H), 1.48 (d, J=7.2 Hz, 3H), and 1.22 (t, J=7.0 Hz, 3H).

Step B: Preparation of ethyl 2-(3-bromo-2-thienylthio)-5-methoxy-2-methylbutyrate

A solution of ethyl 2-(3-bromo-2-thienylthio) propionate (45 g, 152 mmol) in 100 mL DMF was added to a suspension of sodium hydride (6.1 g of a 60% dispersion, 152 mmol) and methoxyethylbromide (27.5 g, 197 mmol) in 400 mL DMF at 5°C. The reaction was allowed to warm to room tempeature and was stirred at room temperature for 5 hours. The reaction was then poured carefully into a mixture of brine (1L) and ethyl acetate (1L). The layers were separated and the aqueous phase was extracted with several additional (220 mL) portions of ethyl acetate. The combined organic phases were dried over anhydrous magnesium sulfate, filtered and concentrated at reduced pressure to give 49.3 g of product.
$^1H$ NMR ($CDCl_3$) δ: 7.45 (d, J=5.6 Hz, 1H), 7.07 (d, J=5.6 Hz, 1H), 4.18 (m, 2H), 3.50 (m, 2H), 3.30 (s, 3H(, 2.40-2.0 (m, 2H), 1.50 (s, 3H) and 1.26 (t, J=1 Hz, 3H).

Step C: Preparation of 2-(3-bromo-2-thienylthio)-4-methoxy-2-methylbutyric acid

The title compound was prepared in 87% yield (3.93 g) from ethyl 2-(3-bromo-2-thienylthio)-4-methoxy-2-methylbutyrate (49 g. 138.6 mmol) in the manner described for the preparation of the product of Example 1, Step C.
$^1H$ NMR ($CDCl_3$) δ: 10.9 (br, 1H), 7.46 (d, J=5.6 Hz, 1H), 7.07 (d, J=5.6 Hz, 1H), 3.57 (t, J=6.5 Hz, 2H), 3.32 (s, 3H), 2.40-3.20 (m, 1H), 2.10-2.00 (m, 1H) and 1.51 (s, 3H).

Step D: Preparation of 2-(3-bromo-2-thienylthio)-4-methoxy-2-methyl-N-methoxy-N-methyl butyramide

The title compound was prepared in 47% yield (18.65 g) from 2-(3-bromo-2-thienylthio)-4-methoxy-2-methyl butyric acid (35 g, 105 mmol) in the manner described for the preparation of the product of Example 1, Step D.
$^1H$ NMR ($CDCl_3$) δ: 7.42 (d, J=5.6 Hz, 1H), 7.05 (d, J=5.6 Hz, 1H), 3.86 (s, 3H), 3.58-3.40 (m, 2H), 3.29 (s, 3H), 3.24 (s, 3H), 2.61-2.50 (m, 1H) 2.09-2.00 (m, 1H) and 1.46 (s, 3H).

Step E: Preparation of 4,5-dihydro-4H-5-(2-methoxyethyl)-5-methyl-4-oxothieno[2,3-b]thiophene

The title compound was prepared in 92% yield (9.87 g) from the butyramide, 18 (18.6 g, 50.5 mmol), in the manner described for the preparation of the product of Example 1, Step E.
$^1H$ NMR ($CDCl_3$) δ: 7.09 (s, 2H), 3.50 (m, 2H), 3.22 (s, 3H), 2.40-23 (m, 1H), 2.11-2.00 (m, 1H) and 1.63 (s, 3H).

Step F: Preparation of 4,5-dihydro-4H-5-(2-methoxyethyl)-5-methyl-4-oxothieno[2,3-b]thiophene-2-sulfonamide

The title compound was prepared in 76% yield (8.1 g) from 4,5-dihydro-4H-5(2-methoxy ethyl)-5 methyl-4-oxo- thieno[2,3-b]thiophene (8.8 g, 38.6 mmol in a manner identical to that already described for the preparation of the product of Example 1, Step F.
$^1H$ NMR (DMSO) δ 7.82 (s, zH), 7.47 (s, 1H), 3.42 (m, 2H), 3.09 (s, 3H), 2.25-2.00 (m, 2H), 1.54 (s, 3H).

Step G: Preparation of 4-acetamido-4,5-dihydro-4H-5-(2-methoxyethyl)-5-methyl thieno[2,3-b]thiophene-2-sulfonamide, α & β-isomer

Employing the procedure described in Example 1, Step G there was prepared the title compound in 73% yield as a 1:1 mixture, of cis & trans isomers from 4,5-dihydro-4H-5-(2-methoxyethyl)-5-methyl-4-oxo-thieno[2,3-b]thiophene-2-sulfonamide (4 g, 14.5 mmol). Chromatography on silica gel using 3% MeOH/$CHCl_3$ as eluent gave 1.2 g pure α isomer and 988 mg pure β isomer.
α isomer:
$^1$H NMR (DMSO) δ: 8.38 (d, J=9 Hz, 1H) 7.58 (s, 2H) 7.29 (s, 1H) 5.06 (d, J=9 Hz, 1H) 3.50 (m, 2H), 3.23 (s, 3H) 2.20-2.10 (m, 1H) 1.90-1.80 (m, 1H) 1.87 (s, 3H) 1.53 (s, 3H).
β-isomer: $^1$H NMR (DMSO) δ: 8.41 (d, J=9 Hz, 1H) 7.59 (s, 2H) 7.24 (s, 1H) 5.26 (d, J=9 Hz, 1H) 3.60-3.50 (m, 2H) 3.20 (s, 3H) 2.20-2.08 (m, 1H) 2.05-1.90 (m, 1H), 1.91 (s, 3H), 1.39 (s, 3H).

Step H: Preparation of 4-acetamido-4,5-Dihydro-4H-5-(2-methoxy-ethyl)-5-methyl thieno[2,3-b]thiophene-2-sulfonamide-6,6-dioxide α & β-isomers

The α isomer of the title compound was prepared in 95% yield (1.2 g) from the α-isomer of Step G by a procedure identical to that already described for the preparation of the product of Example 1, Step H.

The β-isomer of the title compound was prepared in an analogous fashion from the β-isomer of Step G (980 mg, 2.96 mmol). Utilizing the procedure substantially as described for the preparation of the product of Example 1, Step H, there was obtained 720 mg of product (69%) analytical data for α-isomer:
$^1$H NMR (DMSO) δ: 8.60 (d, J=8.8Hz, 1H) 8.10 (s, 2H) 7.54 (s, 1H) 5.32 (d, J=8.8 Hz, 1H) 3.47 (t, J=7.0 Hz, 2H) 3.22 (s, 3H) 2.22-2.10 (m, 1H) 2.00-1.85 (m, 1H) 1.92 (s, 3H) 1.46 (s, 3H)
β-isomer:
$^1$H NMR (DMSO) δ: 8.66 (d, J=9.1 Hz, 1H) 8.10 (s, 2H) 7.53 (s, 1H) 5.49 (d, J=9.1 Hz, 1H) 3.53-3.41 (m, 2H) 3.20 (s, 3H) 2.30-3.19 (m, 1H) 2.08 1,95 (m, 1H) 1.96 (s, 3H) 1.30 (s, 3H)

Step I: Preparation of 4-Ethylamino-4,5-Dihydro-4H-5(2-methoxyethyl)-5-methylthieno[2,3-b]thiophene-6,6-dioxide-2-sulfonamide hydrochloride α & β isomers

The α-isomer of the title compound was prepared from the α-isomer of Step H by a procedure identical to that already described for the prep. of the compound of Example 1, Step I.

The β-isomer of the title compound was prepared from the β-isomer of Step H by an identical procedure.
Analytical Data for:
α-isomer: MP = 217 - 219, $^1$H NMR (DMSO) δ: 9.65 (brs, 1H) 2.22 (brs. 1H) 8.22 (s, 2H) 7.91 (s, 1H) 4.89 (brs, 1H) 3.8-3.55 (m, 2H) 3.30 (s, 3H) 3.15-2.85 (m, 2H) 2.70-2.49 (m, 1H) 2.30-2.18 (m, 1H) 1.49 (s, 3H) 1.27 (t, J=7.1 Hz, 3H): Analysis calcd. for $C_{12}H_{20}M_2O_5$:

| | | C | H | N |
|---|---|---|---|---|
| HCl | Calcd. | 35.58 | 5.22 | 6.91 |
| | Found | 35.70 | 5.16 | 6.93 |

β-isomer: MP = 218 - 220; $^1$H NMR (DMSO) δ: 10.0 (brs, 1H) 9.88 (brs, 1H) 8.22 (s, 2H) 7.94 (s,1H) 5.10 (brs, 1H) 3.90-3.70 (m, 1H) 3.60-3.40 (m, 2H) 3.22 (s, 3H) 3.20-3.05 (m, 1H) 2.35-2.20 (m, 2H) 1.65 (s, 3H) 1.31 (t, J=6.8 Hz, 3H): Analysis calcd. for $C_{12}H_{20}N_2O_5S_3$:

| | C | H | N |
|---|---|---|---|
| Calcd. | 35.58 | 5.22 | 6.91 |
| Found | 35.90 | 5.19 | 6.93 |

Example 4

4,5-Dihydro-5-methyl-5-(2-methoxy)ethyl-4-propylamino-4H-thieno[2,3-b]thiophene-2-sulfonamide α-isomer

Step A: Preparation of 4,5-Dihydro-5-methyl-5-(2-methoxyethyl-4-propionamido-4H-thieno[2,3-b]thiophene-2-sulfonamide α & β-isomers:

To a mixture of sodium borohydride (0.64 g, 16.9 mmol) in absolute ethanol (25 ml) at 0°C was added the ketone 20 (3.41 g, 11.1 mmol) in tetrahydrofuran (60 ml) and the resulting mixture was stirred with warming to room temperature for 3 hours. The reaction was quenched with water and 1 N HCl and the aqueous was made basic with sat. sodium bicarbonate solution. The aqueous was extracted with ethyl acetate (3 x 100 ml). The combined extracts were dried over $MgSO_4$ and concentrated to yield 3.48 g (>100%) of the crude alcohol 21 as a white foam. To a solution of the crude alcohol 21 (3.32 g, 10. 7 mmol) in propionitrile (20 ml) at 0°C was added concentrated $H_2SO_4$ (0.60 ml). After 2 hours, the reaction mixture was warmed to room temperature and quenched with 10 N NaOH and sodium bicarbonate. The aqueous was extracted with ethyl acetate (3 x 100 ml) and the combined extracts were dried over $MgSO_4$ and concentrated. The product was purified by flash chromatography with silica gel and 35-50% ethyl acetate/hexane as eluant to yield 1.343 g (34.3%) of the amide α-isomer:
$^1$H NMR (δ from TMS in DMSO-$d_6$ in ppm): 8.32 (d, 1H, J=9.0 Hz), 7.59 (s, 2H), 7.28 (s, 1H), 5.08 (d, 1H, 9.0 Hz), 3.48-4.30 (m, 2H), 3.23 (s, 3H), 2.19-2.11 (m, 2H), 1.87-1.82 (m, 1H), 1.53 (s, 3H), 1.40 (m, 1H), 1.01 (t, 3H, J=7.6 Hz) and 0.58 g (14.8%) of the amide β-isomer:
$^1$H NMR (δ from TMS in DMSO-$d_6$ in ppm: 8.34 (d, 1H, J=9.0 Hz), 7.58 (s, 2H), 7.22 (s, 1H), 5.27 (d, 1H, J=9.0 Hz), 3.49-4.44 (m, 2H), 3.20 (s, 3H), 2.22-2.12 (m, 3H), 2.00-1.98 (m, 1H), 1.38 (s, 3H), 1.02 (t, 3H, J=7.6 Hz).

Step B: Preparation of 4,5-Dihydro-5-methyl-5-(2-methoxy)ethyl-4-propylamino-4H-thieno[2,3-b]thiophene-2-sulfonamide:

To a solution of the α-isomer product of Example 4, step A (0.399 g, 1.09 mmol) in tetrahydrofuran (15 ml) was added $BH_3$ (0.35 ml, 3.5 mmol) as a 10 M borane-dimethylsulfide complex solution. The resulting solution was stirred at room temperature for 20 minutes and heated to 60-64°C for 2 hours. More $BH_3$ (0.20 ml, 2.0 mmol) was added and the solution heated for 1 hour, cooled to room temperature, and stirred overnight, about 16 hours. The reaction was quenched with ethanol followed by water and 1 N HCl. When $H_2$ gas evolution ceased, the reaction mixture was heated on a steam bath for 15 minutes, cooled to room temperature, and made neutral with 10 N NaOH and $NaHCO_3$. The aqueous solution was extracted with ethyl acetate (3 x 50 ml) and the combined extracts were dried over $MgSO_4$ and concentrated. The product was purified by flash chromatography with silica gel and 1% methanol/methylene chloride as eluant. The product was dissolved in absolute ethanol and 1 eq. of HCl as a 3.22 M ethanolic HCl solution was added to the solution. The product was allowed to crystallize to yield 0.083 g (21.7%): mp = 169-172°C with decomposition;
$^1$H NMR (δ from TMS in DMSO-$d_6$ in ppm): 9.18-9.05 (m, 2H), 7.72 (s, 2H), 7.64 (s, 1H), 4.45 (d, 1H, J=7.7Hz), 3.61-3.55 (m, 2H), 3.43-3.38 (m, 1H), 3.31 (s, 3H), 2.85-2.72 (m, 2H), 2.36-2.28 (m, 1H), 1.80-1.75 (m, 1H), 1.64-1.60 (m, 1H), 1.54 (s, 3H), 0.90 (t, 3H, J=7.3 Hz); Anal. Calcd. for $C_{13}H_{22}N_2S_3O_3 \cdot HCl$:

| | |
|---|---|
| | C, 40.35; H, 5.99; N, 7.24. |
| Found: | C, 40.42; H, 5.67; N, 7.19. |

Example 5

4,5-Dihydro-5-methyl-5-(2-methoxy)ethyl-4-propylamino-4H-thieno[2,3-b]thiophene-2-sulfonamide-6,6-dioxide

Step A: Preparation of 4,5-Dihydro-5-methyl-5-(2-methyl)-ethyl-4-propionamido-4H-thieno[2,3-b]thiophene-2-sulfonamide-6,6-dioxide α-isomer:

To a solution of the α-isomer product of Example 4, Step B (0.63 g, 1.73 mmol), in methanol (15 ml) and water (10 ml) was added oxone (2.14 g, 3.48 mmol) as a solution in water (20 ml). Upon addition of the oxone to the methanol, the oxone fell out of solution. The reaction mixture was allowed to stir overnight, about 16 hours. The reaction was quenched with aqueous sat. $NaHCO_3$ and extracted with ethyl acetate (3 x 50 ml). The combined extracts were dried over $MgSO_4$ and concentrated. The product was purified by flash chromatography with silica gel and eluted with 2-5% methanol/methylene chloride to yield 0.529 g (77.2%) of the α-isomer product.
$^1H$ NMR (δ from TMS in DMSO-$d_6$ in ppm): 8.52 (d, 1H, J=8.7 Hz), 8.07 (s, 2H), 7.51 (s, 1H), 5.31 (d, 1H, J=8.7 Hz), 3.44 (t, 2H, J=7.1 Hz), 3.19 (s, 3H), 2.21-2.11 (m, 3H), 1.89-1.84 (m, 1H), 1.43 (s, 3H), 1.00 (t, 3H, J=7.5 Hz).

Step B: Preparation of 4,5-Dihydro-5-methyl-5-(2-methoxy)-ethyl-4-propionamido-4H-thieno[2,3-b]thiophene-2-sulfonamide-6,6-dioxide β-isomer:

To a solution of the β-isomer product of Step A (0.44 g, 1.21 mmol) in methanol (15 ml) and water (10 ml) was added oxone (1.51 g, 2.46 mmol) as a solution in water (20 ml). Upon addition of the oxone to the methanol, the oxone fell out of solution. The reaction mixture was allowed to stir overnight, about 16 hours. The reaction was quenched with aqueous sat. $NaHCO_3$ and extracted with ethyl acetate (3 x 50 ml). The combined extracts were washed with sat. brine (50 ml) and dried over $MgSO_4$ and concentrated to yield 0.431 g (89.9%) of product:
$^1H$ NMR (δ from TMS in DMSO-$d_6$ in ppm): 8.58 (d, 1H, J=9.0 Hz), 8.10 (s, 2H), 7.50 (s, 1H), 5.50 (d, 1H, J=9.0 Hz), 3.51-3.34 (m, 2H), 3.20 (s, 3H), 2.28-2.25 (m, 3H), 2.10-2.00 (m, 1H), 1.30 (s, 3H), 1.06 (t, 3H, J=7.6 Hz).

Step C: Preparation of 4,5-Dihydro-5-methyl-5-(2-methoxy)-ethyl-4-propylamino-4H-thieno[2,3-b]thiophene-2-sulfonamide-6,6-dioxide α-isomer:

To a solution of the α-isomer product of Step A (0.470 g, 1.18 mmol) in tetrahydrofuran (20 ml) was added $BH_3$ (0.40 ml, 4.0 mmol) as a 10 M borane- dimethylsulfide complex solution. The resulting solution was heated to 60-64°C for 2 hours. More of the $BH_3$ (0.20 ml, 2.0 mmol) was added and the solution heated for 1 hour. The reaction mixture was cooled to room temperature and quenched with ethanol followed by water and 1 N HCl. When $H_2$ gas evolution ceased, the reaction mixture was heated on a steam bath for 15 mintues, cooled to room temperature, and made neutral with 10 N NaOH and $NaHCO_3$. The aqueous solution was extracted with ethyl acetate (4 x 50 ml) and the combined extracts were washed with sat. brine (50 ml), dried over $MgSO_4/NaHCO_3$, and concentrated. The product was purified by flash chromatography on silica gel and 0.5-1.5% methanol/methylene chloride as eluant to yield an off-white oil. The product was dissolved in absolute ethanol and 1 eq. of HCl as a 3.22 M ethanolic HCl solution was added to the solution. The product was allowed to crystallize from diethyl ether/ethanol to yield 0.211 g (42.7%) of product: mp=185-189°C with decomposition;
$^1H$ NMR (δ from TMS in DMSO-$d_6$ in ppm): 9.90 (bs, 1H), 9.30 (bs, 1H), 8.24 (s, 2H), 7.96 (s, 1H), 4.89 (bm, 1H), 3.62 (m, 2H), 3.29 (s, 3H), 2.96-2.92 (m, 1H), 2.80-2.75 (m, 1H), 2.65-2.62 (m, 1H), 2.25-2.20 (m, 1H), 1.74-1.64 (m, 2H), 1.50 (s, 3H), 0.91 (t, 3H, J=7.4 Hz);

Anal. Calcd. for $C_{13}H_{22}N_2S_3O_5 \cdot HCl$:
C, 37.26; H, 5.53; N, 6.69.
Found: C, 37.50; H, 5.28; N, 6.60.

Step D: Preparation of 4,5-Dihydro-5-methyl-5-(2-methoxy)-ethyl-4-propylamino-4H-thieno[2,3-b]thiophene-2-sulfonamide-6,6-dioxide β-isomer:

To a solution of the product of Step B (0.3757 g, 0.948 mmol) in tetrahydrofuran (20 ml) was added $BH_3$ (0.35 ml, 3.5 mmol) as a 10 M borane-dimethylsulfide complex solution. The resulting solution was heated to 60-64°C for 1 hour. More of the $BH_3$ (0.15 ml, 1.5 mmol) was added and the solution heated for 1 hour. The reaction mixture was cooled to room temperature and quenched with ethanol followed by water and 1 N HCl. When $H_2$ gas evolution ceased, the reaction mixture was heated on a steam bath for 15 minutes, cooled to room temperature, and made neutral with 10 N NaOH and $NaHCO_3$. The aqueous solution was extracted with ethyl acetate (4 x 50 ml) and the combined extracts were washed with sat. brine (50 ml), dried over $MgSO_4/NaHCO_4$, and concentrated. The product was purified by flash chromatography with silica gel and 0.5-1.5% methanol/methylene chloride as eluant to yield 0.2855 g (78.7%) of the free amine as an off-white solid. The product was dissolved in absolute ethanol and 1 eq. of HCl as a 3.22 M ethanolic HCl solution was added to the solution. The product was allowed to crystallize from diethyl ether/ethanol to yield 0.200 g (50.4%) of product: mp=134.5-136°C with decomposition;
$^1H$ NMR (δ from TMS in DMSO-$d_6$ in ppm): 10.12 (bs, 1H), 9.90 (bs, 1H), 8.25 (s, 2H), 8.00 (s, 1H), 5.12 (bm,

1H), 3.58-3.50 (m, 2H), 3.24 (s, 3H), 3.15-3.12 (m, 1H), 3.10-2.96 (m, 1H), 2.31-2.27 (m, 2H), 1.84-1.74 (m, 2H), 1.66 (s, 3H), 0.95 (t, 3H, J=7.3 Hz);

Anal. Calcd. for $C_{13}H_{22}N_2S_3O_5$•HCl•$C_2H_6O$:
C, 38.74; H, 6.26; N, 6.02.
Found: C, 38.66; H, 5.94; N, 6.04.

Example 6

4,5-Dihydro-4-ethylamino-4H-5-(3-methoxypropyl)-5-methylthieno[2,3-b]thiophene-2-sulfonamide 6,6-dioxide

Step A: Preparation of Dimethyl-2-(3-Bromo-2-thienylthio)glutarate

A solution of 2,3-Dibromothiophene (50 g, 206 mmol) in ether (1L) at -70°C was treated with n-butyl lithium (82 ml of 2.5 N solution in hexanes, 206 ml). The solution was stirred at -70°C for 5 minutes and sulfur was added in one portion 6.61 g (206 mm). The reaction mixture was warmed to -40°C until all of the solids dissolved and then cooled to -70°C. At this time dimethyl-2-bromoglutarate was added and the reaction warmed slowly to room temperature. After stirring at room temperature overnight the reaction was poured into 2L saturated sodium bicarbonate solution. The layers were separated and the aqueous phase was extracted with ethyl acetate. The combined organic layers were dried over $MgSO_4$, filtered, and concentrated at reduced pressure. The residue was chromatographed on silica gel using 5-10% ethyl acetate/hexane as eluent to give 49 g of product:
$^1H$ NMR $CDCl_3$ δ:7.40 (d, J=5.6 Hz, 1H), 7.00 (d, J=5.6 Hz, 1H), 3.70 (s, 3H), 3.62 (t, J=7 Hz, 1) 2.7-2.5 (m, 2H), 2.5-2.0 (m, 2H).

Step B: Preparation of Dimethyl-2-(3-bromo-2-thienylthio)-2-methylglutarate

A solution of Dimethyl-2-(3-bromo-2-thienylthio) glutarate (29) (49 g, 152.5 mmol) in THF (100 ml) was added slowly to a stirring suspension of sodium hydride (6.06 g of a 60% disp. 152 mmole) and iodomethane (43 g, 302 mmol) in THF (400 ml) at room temperature. The reaction was stirred overnight at room temperature and then poured into 2L saturated sodium bicarbonate. Ethyl acetate (1L) was then added and the layers were separated. The aqueous phase was extracted with ethyl acetate and the combined organic layers were dried over $MgSO_4$, filtered and concentrated in vacuo. The residue was chromatographed on silica gel using 5-10% ethyl acetate/hexane as eluent to give 32.5 g of product:
$^1H$ NMR $CDCl_3$ δ: 7.46 (d, J=5.5 Hz, 1H), 7.07 (d, J=5.5 Hz, 1H), 3.73 (s, 31), 3.68 (s, 3H), 2.6-2.1 (m, 4H), 1.46 (s, 3H).

Step C: Preparation of 4-(3-bromo-2-thienylthio)-4-carbomethoxy-pentanoic acid

A solution of Dimethyl-2-(3-bromo-2-thienylthio)-2-methyl glutarate (31 g, 84 mmol) in methanol (400 mL) was treated with a solution of NaOH (1 M) in water. The reaction was stirred for 1 hour at room temperature. The reaction was then concentrated to 1/4 volume in vacuo and poured into 1L $H_2O$. The pH was adjusted to ~12 and the aqueous mixture was extracted with ethyl acetate (200 mL). The pH was then adjusted to pH 2 with 6 N HCl and the mixture was extracted repeatedly with ethyl acetate. The acidic extracts were combined, dried over anhydrous $MgSO_4$, filtered and concentrated at reduced pressure to give an essentially quantitative yield of product:
$^1H$ NMR $CDCl_3$ δ: 7.48 (d, J=5.5 Hz, 1H), 7.10 (d, J=5.5 Hz, 1H), 3.70 (s, 3H), 2.7-2.1 (m, 4H), 1.5 (s, 3H).

Step D: Preparation of Methyl-2-(3-bromo-2-thienylthio)-2-methyl-5-hydroxy valerate

A solution of 4-(3-bromo-2-thienylthio)4-carbomethoxy-pentanoic acid (28 g, 79 mmol) in THF (400 mL) was cooled to 0°C under 1 atm. of $N_2$ and treated with $BH_3$·DMS (8 ml, 80 mmol) and warmed to room temperature. The reaction was stirred at room temprature for 2 hours and then quenched by the careful addition of 1 N HCl (50 mL). The reaction mixture was then poured into 500 mL 1 N HCl and extracted repeatedly with ethyl acetate. The combined organics were washed once with saturated sodium bicarbonate, dried over anhydrous magnesium sulfate, filtered and concentrated in vacuo to give 22 g of product:
$^1H$ NMR $CDCl_3$ δ: 7.45 (d, J=5.5 HZ, 1H), 7.05 (d, J=5.5 Hz, 1H), 3.74 (s, 3H), 3.66 (t, J=65, 2H), 2.1-1.5 (m,

4H), 1.49 (s, 3H).

Step E: Preparation of Methyl-2-(3-bromo-2-thienylthio)-5-methoxyvalerate

A solution of methyl 2-(3-bromo-2-thienylthio)-2-methyl-5-hydroxyvalerate (21.2 g, 88.6 mmol) in ether (300 mL) was treated with silver (I) oxide (26 g, 112 mmole) and iodomethane (44.6 g, 300 mmol) at room temperature. The reaction mixture was stirred at room temperature for 24 hours at which time additional portions of silver (I) oxide (25 g, 107 mmol) and iodomethane (45.6 g, 320 mmol) were added. The reaction mixture was stirred at room temperature for 48 hours and filtered. The solvent was evaporated in vacuo, and the residue was chromatographed on silica gel to give (15% ethyl acetate/hexanes) 16.5 g (73%) of product as an oil:
$^1$H NMR $CDCl_3$ δ: 7.45 (d, J=5.6 Hz, 1H), 7.06 (d, J=5.6 Hz, 1H), 3.73 (s, 3H), 3.39 (t, J=6.5 Hz, 2H), 3.32 (s, 3H), 2.1-1.4 (m, 4H), 1.48 (s, 3H).

Step F: Preparation of 2-(3-bromo-2-thienylthio)-2-methyl-5-methoxy valeric acid

The title compound was prepared from methyl 2-(3-bromo-2-thienylthio)-2-methyl-5-methoxy-valerate (33 g, 93 mmol) in a manner analogous to that already described in Example 1, Step C. There was obtained 29.2 g (92%) as an oil:
$^1$H NMR $CDCl_3$: δ 7.46 (d, J=5.5 Hz, 1H), 7.08 (d, J=5.5 Hz, 1H), 3.42 (t, J=6.3 Hz, 2H), 3.34 (s, 3H), 2.1-1.6 (m, 4H), 1.47 (s, 3H).

Step G: Preparation of 2-(3-bromo-2-thienylthio)-5-methoxy-2-methyl-N-methyl-N-methoxy valeramide

A solution of 2-(3-bromo-2-thienylthio)-2-methoxy-2-methylvaleric acid (28 g, 82 mmole) in DMF (200 ml) was treated with carbonyl diimidazole (20 g, 123 mmole) and after 10 minutes with N,O-Dimethyl hydroxylamine hydrochloride (24 g, 246 mmol), and N-methyl piperidine. The reaction was stirred overnight at room temperature (18 hours), poured into about 1L of saturated sodium bicarbonate and extracted with ethyl acetate. The organic extracts were dried over magnesium sulfate, filtered and concentrated to give 18 g product as an oil:
$^1$H NMR ($CDCl_3$) δ: 7.40 (d, J=5.6 Hz, 1H), 7.04 (d, J=5.6 Hz, 1H), 3.86 (s, 3H), 3.38 (t, J=6.3 Hz, 2H), 3.32 (s, 3H), 3.27 (s, 3H), 2.30-2.18 (m, 1H), 1.80-1.40 (m, 3H), 1.43 (s, 3H).

Step H: Preparation of 5-(3-methoxypropyl)-5-methyl-4,5-dihydro-4H-4-oxo-thieno[2,3-b]thiophene

The title compound was prepared from the product of Step G (22.5 g, 58.8 mmol) in a manner identical to that described in Example 1, Step E, to give 13.782 g (96%) as an oil:
$^1$H NMR ($CDCl_3$) δ: 7.17 (d, J=5.3 Hz, 1H), 7.14 (d, J=5,3 Hz, 1H), 3.34 (t, J=6.2 Hz, 2H), 3.29 (s, 3H), 2.09-1.62 (m, 3H), 1.62 (s, 3H), 1.58-1.40 (m, 1H).

Step I: Preparation of 4,5-Dihydro-5-(3-methoxypropyl)-5-methyl-4-O-4-H-thieno[2,3-b]thiophene-2-sulfonamide

The title compound was prepared in 65% yield from the product of Step H in a manner analogous to that described in Example 1, Step F,
mp = $^1$H NMR (DMSO) δ: 7.8 (brs, 2H), 7.48 (s, 1H), 3.27(t, J=6.1 Hz, 2H), 3.17 (s, 3H), 1.95-1.85 (m, 2H), 1.62-1.55 (m, 1H), 1.54 (s, 3H).

Step J: Preparation of 4,5-Dihydro-4H-4-ethylamino-5-(3-methoxypropyl)5-methyl-thieno[2.3-b]thiophene-2-sulphonamide

A solution of 4,5-Dihydro-4H-5-(3-methoxypropyl)5-methyl-4-oxo-thieno[2,3-b]thiopyran (37) (2.1 g, 6.5 mmol) in 50 ml of 1/1 THF/benzene at ambient temperature was treated with titanium tetrachloride (1.2 g, 716 mL, 6.5 mmol) and then ethylamine (2.9 g, 65 mmol). The reaction was stirred at room temperature for about 5 hours until TLC analysis showed the absence of starting material. The reaction mixture was then poured into saturated sodium bicarbonate solution and extracted with ethyl acetate. The ethyl acetate layers were dried over magnesium sulfate, filtered and concentrated at reduced pressure. The residue was dissolved in ethanol (50 mL) and treated with excess sodium borohydride for 3 hours. The reaction was then poured into saturated sodium bicarbonate and extracted with ethyl acetate. The organic extracts were dried over magnesium sulfate, filtered and concentrated in vacuo. The residue was chromatographed on silica gel using 3% methanol/ chloroform as eluent to give 1.1 g of α isomer and 310 mg of β isomer.
α isomer: $^1$H NMR (DMSO) δ: 7.58 (s, 2H), 7.40 (s, 1H), 3.8 (brs, 1H), 3.35 (m, 3H), 3.20 (s, 3H), 2.75-2.50 (m, 2H), 2.05-1.40 (m, 4H), 1.52 (s, 3H), 1.03 (t, J=7.1 Hz, 3H)
β isomer: $^1$H NMR (DMSO) δ: 7.55 (s, 2H), 7.38 (s, 1H), 3.78 (brs, 1H), 3.35 (m, 3H), 3.22 (s, 3H), 2.80-2.50 (m, 2H), 2.10-1.50 (m, 4H), 1.49 (s, 3H), 1.01 (appt, J=7.1 Hz, 3H).

Step K: Preparation of 4,5-Dihydro-4H-4-ethylamino-5-(3-methoxypropyl)-5-methyl-thieno[2,3-b]thiophene-6,6-dioxide-2-sulfonamide β-isomer

A solution of 4,5-Dihydro-4H-4-ethylamino-5-(3-methoxypropyl)-5-methylthieno[2,3-b]thiophene-2-sulfonamide (400 mg, 14 mmol) in 20 ml methanol was treated with a solution of oxone® (1.2 g, 1.9 mmol) in 20 ml water. The reaction was stirred at room tempeature for 24 hours, poured into 100 ml saturated sodium bicarbonate and extracted with ethyl acetate. The ethyl acetate extracts were extracted with 1 N HCl (3 x 50 ml) and discarded. The acidic aqueous extracts were basified to pH 9 and extracted with ethyl acetate. The solvent was evaporated at reduced pressure and the residue was dissolved in ethanol. A solution of excess HCl in ethanol was then added and the solvent was evaporated to give a white solid. The solid was recrystallized from ethanol to give 47 mg of product mp=230°C.

Elemental analysis calc'd for $C_{13}H_{22}N_2O_5S_3$•HCl

| | C | H | N |
|---|---|---|---|
| Calc'd | 37.26 | 5.53 | 6.68 |
| Found | 37.47 | 5.51 | 6.72 |

EXAMPLE 7

4,5-Dihydro-4-hydroxy-5-propyl-4H-thieno[2,3-b]thiophene-2-sulfonamide-6,6-dioxide

Step A: Preparation of 2-(3-Bromo-2-thienylthio)valeric acid:

To a solution of the ester product of Example 1, Step A (20.0 g, 61.9 mmol) in methanol (200 ml) was added sodium hydroxide (75 ml, 750 mmol) as a 10 N aqueous solution and the resulting solution was heated to reflux for 4 hours. ThE reflux condensor was removed and a portion of the methanol was boiled away. The product mixture was poured into a beaker containing concentrated HCl and ice (200 g) to make an aqueous solution with pH < 7. The aqueous was extracted with ethyl acetate (3 x 200 ml) and the combined extracts were dried over $MgSO_4$ and concentrated to yield 18.2 g (99.6%) of product as a yellow oil: (δ from TMS in $CDCl_3$ in ppm): 9.70 (bs, 1H), 7.40 (d, 1H, J=5.5 Hz), 7.04 (d, 1H, J=5.5 Hz), 3.56 (t, 1H, J=8.0 Hz), 1.88-1.73 (m, 2H), 1.57-1.48 (m, 2H), 0.96 (t, 3H, J=7.3 Hz).

Step B: Preparation of 2-(3-Bromo-2-thienylthio)-N-methyl-N-methoxy-valeramide:

To a solution of the product from Step A (16.1 g, 54.5 mmol) in N,N′-dimethylformamide (65 ml) was added, portionwise at room temperature, N,N′-carbonyldiimidazole (CDI) (17.75 g, 109.5 mmol) and the resulting solution was stirred for 0.5 hours or until $CO_2$ evolution could no longer be observed. This solution was then added to a stirring mixture of N,O-dimethylhydroxylamine hydrochloride (11.45 g, 110.3 mmol) in N,N′(-dimethylformamide (70 ml). The resulting mixture was stirred for 15 hours and then was diluted with water and $NaHCO_3$ solution. The aqueous was extracted with ethyl acetate (4 x 100 ml). The combined extracts were washed with 1N HCl (20 x 50 ml) and then with sat. $NaHCO_3$ (2 x 50 ml) and dried over $MgSO_4$/$NaHCO_3$ and concentrated to yield 21.3 g of a yellow oil. The product was purified by flash chromatography with silica gel and 15-40% ethyl acetate/hexane as eluant to yield 14.66 g (79.5%) of product as a yellow oil: $^1$H NMR (δ from TMS in $CDCl_3$in ppm): 7.38 (d, 1H, J=5.5 Hz), 7.03 (d, 1H, J=5.5 Hz), 4.08 (t, 1H, J=7.3 Hz), 3.62 (s, 3H), 3.18 (s, 3H), 1.93-1.74 (m, 2H), 1.48-1.39 (m, 2H), 0.93 (t, 3H, J=7.3 Hz). $^{13}$C NMR (δ $CDCl_3$ in ppm): 171.74, 130.87, 130.69, 127.75, 120.92, 61.46, 47.90, 33.64, 32.47, 20.54, 13.77.

Step C: Preparation of 4,5-Dihydro-5-propyl-4-oxo-4H-thieno[2,3-b]thiophene-6,6-dioxide (**43**):

To a solution of the amide sulfide **41** (5.10 g, 15.1 mmol) in anhydrous diethyl ether (150 ml) was added, at -78°C over 15 minutes, n-butyllithium (9.00 ml, 22.5 mmol) as a 2.5 M solution in hexanes. The resulting solution was allowed to stir for 0.75 hours. The dry ice bath was removed and the reaction was quenched with aqueous sat., $NaHCO_3$. The layers were separated and the aqueous was extracted with ethyl acetate (2 x 100 ml) and with diethyl ether (100 ml). The combined extracts were dried over $MgSO_4/NaHCO_3$ and concentrated to yield 3.0 g (> 100%) of the ketone **42**. To a solution of the ketone **42** (2.25 g, 11.4 mmol) in methanol (150 ml) was added oxone (14.0 g, 22.8 mmol) and $NaHCO_3$ (1.66 g, 19.8 mmol) in water (150 ml). The oxone fell out of solution upon addition to the methanol solution. The reaction mixture was allowed to stir at room temperature for 16 hours. The product mixture was diluted with water and sat., $NaHCO_3$ and the aqueous was extracted with ethyl acetate (5 x 100 ml). The combined extracts were dried over $MgSO_4/NaHCO_3$ and concentrated. The product was purified by flash chromatography with silica gel and 10-20% ethyl acetate-/hexane as eluant to yield 1.46 g of **43** (55.9%) as a pale, viscous oil: $^1H$ NMR (δ from TMS in $CDCl_3$ in ppm): 7.84 (d, 1H, J=5.0 Hz), 7.37 (d, 1H, J=5.0 Hz), 4.23 (dd, 1H, J=7.5 Hz), 2,16 (m, 2H), 1.81-1.69 (m, 2H), 1.08 (t, 3H, J=7.5 Hz).

Step D: Preparation of 4,5-Dihydro-5-propyl-4-oxo-4H-thieno[2,3-b]thiophene-6,6-dioxide (**44**):

To a mixture of sodium borohydride (0.042 g, 0.79 mmol) in ethanol (2 ml) was added the ketone sulfone **43** (0.114 g, 0.495 mmol) in dry tetrahydrofuran (4 ml) and the resulting mixture was allowed to stir at room temperature for 5 hour. The reaction was quenched with water and sat., $NaHCO_3$ and the aqueous was extracted with ethyl acetate (4 x 20 ml). The combined extracts were dried over $MgSO_4$ and concentrated. The product was purified by flash chromatography with silica gel and 20% ethyl acetate/hexane as eluant to yield 0.050 g (43%) of 44α: $^1H$ NMR: (δ from TMS in $CDCl_3$ in ppm): 7.68 (d, 1H, J=4.9 Hz), 7.07 (d, 1H, J=4.9 Hz), 4.90 (dd, 1H, J=8.2 Hz), 3.76-3.69 (m, 1H), 3.46 (d, 1H, J=8.2 Hz), 2.14-2.05 (m, 1H), 1.99-1.87 (m, 1H), 1.71-1.58 (m, 2H), 1.02 (t, 3H, J=7.2 Hz); and 0.050 g (43%) of 44β: $^1H$ NMR: (δ from TMS in $CDCl_3$ in ppm): 7.71 (d, 1H, J=4.9 Hz), 7.10 (d, 1H, J=4.9 Hz), 5.10 (dd, 1H, J=10.0 Hz), 3.91-3.84 (m, 1H), 2.51 (d, 1H, J = 10.0 Hz), 2.08-1.94 (m, 2H), 1.68-1.61 (m, 2H), 1.05 (t, 3H, J=7.3 Hz).

Step E: Preparation of 4,5-Dihydro-4-hydroxy-5-propyl-4H-thieno[2,3-b]thiophene-2-sulfonamide-6,6-dioxide (**45**):

To a solution of the alcohols **44**α and **44**β (0.469 g, 2.02 mmol) in anhydrous tetrahydrofuran (20 ml) at -80°C (diethyl ethyer, dry ice) was added n-butyllithium (1.6 ml, 4.0 mmol) as a 2.5 M solution in hexanes. The resulting orange solution was stirred at -80°C for 1.5 hours. Additional n-butyl-lithum (0.20 ml, 0.50 mmol) was added and the reaction was allowed to continue for 1 hour. This solution was added to a flask which contained anhydrous $SO_2$ in anhydrous tetrahydrofuran (5 ml) at -78°C. The resulting yellow solution was allowed to stir at -78°C for 15 minutes and then was warmed to room temperature for 0.75 hours. The solvent was removed by evaporation in vacuo to yield an orange, viscous oil. This residue was dissolved in $H_2O$ (10 ml) and THF (2 ml) and cooled in an ice bath. To this was added hydroxylamine-O-sulfonic acid (1.24 g, 11.0 mmol) and sodium acetate (1.45 g. 10.7 mmol) and the resulting solution was allowed to stir with warming to room temperature for 1.5 hours. The reaction mixture was diluted with water (pH=4-5) and extracted with ethyl acetate (3 x 30 ml). The aqueous was made neutral with $NaHCO_3$ and was extracted with ethyl acetate (3 x 30 ml). The aqueous was then brought up to pH=8 and extracted with ethyl acetate (2 x 30 ml). The organic extracts were combined, dried over $MgSO_4$ and concentrated. The product was purified by flash chromatography with silica gel and 30-60% ethyl acetate/hexane as eluant to yield 0.084 g (13%) of **45**α: $^1H$ NMR: (δ from TMS in $CDCl_3$ in ppm): 8.12 (s, 2H), 7.57 (s, 1H), 6.55 (bs, 1H), 4.94 (d, 1H, J=5.37 Hz), 3.73 (q, 1H, J=8.55 Hz), 2.02-1.90 (m, 2H), 1.63-1.55 (m, 2H), 0.99 (t, 3H, J=7.38 Hz); and 0.072 g (11%) of **45**β:
$^1H$ NMR: (δ from TMS in $CDCl_3$ in ppm): 8.09 (s, 2H), 7.58 (s, 1H), 6.18 (bs, 1H), 5.16 (d, 1H, J=5.86 Hz), 4.04 (q, 1H, J=6.0 Hz), 1.87-1.79 (m, 2H), 1.56-1.48 (m, 2H), 0.98 (t, 3H, J=7.2 Hz); and 0.151 g (24%) of **45**α and **45**β for a total yield of 48%.

EXAMPLE 8

4,5-Dihydro-5-propyl-4-oxo-4H-thieno-[2,3-b]thiophene-6,6-dioxide

Step A: Preparation of 2-(3-bromo-2-thienylthio)-valeric acid (**40**):

To a solution of ethyl 2-(3-bromo-2-thienylthio)-valerate (**1**) (20.0 g, 61.9 mmol) in methanol (200 ml) was added sodium hydroxide (75 ml, 750 mmol) as a 10 N aqueous solution and the resulting solution was heated to reflux for 4 hours. The reflux condensor was removed and a portion of the methanol was boiled away. The product mixture was poured into a beaker containing concentrated HCl and ice (200 g) to make an aqueous solution with pH < 7. The aqueous was extracted with ethyl acetate (3 x 200 ml) and the combined extracts were dried over $MgSO_4$ and concentrated to yield 18.2 g (99.6%) of 2-(3-bromo-2-thienylthio)-valeric acid (**40**) as a yellow oil:
(δ from TMS in $CDCl_3$ in ppm): 9.70 (bs, 1H), 7.40 (d, 1H, J=5.5 Hz), 7.04 (d, 1H, J=5.5 Hz), 3.56 (t, 1H, J=8.0 Hz), 1.88 - 1.73 (m, 2H), 1.57 - 1.48 (m, 2H), 0.96 (t, 3H, J=7.3 Hz).

Step B: Preparation of 2-(3-bromo-2-thienylthio)-N-ethyl-N-methoxy-valeramide (**41**):

To a solution of 2-(3-bromo-2-thienylthio)-valeric acid (**40**) (16.1 g, 54.5 mmol) in N,N'-dimethylformamide (65 ml) was added, portionwise at room temperature, N,N'-carbonyldiimidazole (17.75 g, 109.5 mmol) and the resulting solution was stirred for 0.5 hour or until $CO_2$ evolution could no longer be observed. This solution was then added to a stirring mixture of N,O-dimethylhydroxylamine hydrochloride (11.45 g, 110.3 mmol) in N,N'-dimethylformamide(70 ml). The resulting mixture was stirred for 15 h and then was diluted with water and $NaHCO_3$ solution. The aqueous was extracted with ethyl acetate (4 x 100 ml). The combined extracts were washed with 1 N HCl (20 x 50 ml) and then with sat. $NaHCO_3$ (2 x 50 ml) and dried over $MgSO_4/NaHCO_3$ and concentrated to yield 21.3 g of a yellow oil. The product was purified by flash chromatography with silica gel and 15% - 40% ethyl acetate/hexane as eluant to yield 14.66 g (79.5%) of 2-(3-bromo-2-thienylthio)-N-methyl-N-methoxy-valeramide (**41**) as a yellow oil:
$^1H$ NMR (δ from TMS in $CDCl_3$ in ppm): 7.38 (d, 1H, J=5.5 Hz), 7.03 (d, 1H, J=5.5 Hz), 4.08 (t, 1H, J=7.3 Hz), 3.62 (s, 3H), 3.18 (s, 3H), 1.93 - 1.74 (m, 2H), 1.48 - 1.39 (m, 2H), 0.93 (t, 3H, J=7.3 Hz); $^{13}C$ NMR (d from $CDCl_3$ in ppm): 171.74, 130.87, 130.69, 127.75, 120.92, 61.46, 47.90, 33.64, 32.47, 20.54, 13.77.

Step C: Preparation of 4,5-Dihydro-5-propyl-4-oxo-4H-thieno-[2,3-b]thiophene-6,6-dioxide (**43**):

To a solution of 2-(3-bromo-2-thienylthio)-N-methyl-N-methoxy-valeramide (**41**) (5.10 g, 15.1 mmol) in anhydrous diethyl ether (150 ml) was added, at -78°C over 15 minutes, n-butyllithium (9.00 ml, 22.5 mmol) as a 2.5 M solution in hexanes. The resulting solution was allowed to stir for 0.75 h. The dry ice bath was removed and the reaction was quenched with aqueous sat. $NaHCO_3$. The layers were separated and the aqueous was extracted with ethyl acetate (2 x 100 ml) and with diethyl ether (100 ml). The combined extracts were dried over $MgSO_4$ /$NaHCO_3$ and concentrated to yield 3.0 g (>100%) of 4,5-dihydro-5-propyl-4-oxo-4H-thieno[2,3-b]thiophene (**42**). To a solution of 4,5-Dihydro-5-propyl-4-oxo-4H-thieno[2,3-b]thiophene (**42**) (2.25 g, 11.4 mmol) in methanol (150 ml) was added oxone (14.0 g, 22.8 mmol) and $NaHCO_3$ (1.66 g, 19.8 mmol) in water (150 ml). The oxone fell out of solution upon addition to the methanol solution. The reaction mixture was allowed to stir at room temperature for 16 hours. The product mixture was diluted with water and sat. $NaHCO_3$ and the aqueous was extracted with ethyl acetate (5 x 100 ml). The combined extracts were dried over $MgSO_4/NaHCO_3$ and concentrated. The product was purified by flash chromatography with silica gel and 10% - 20% ethyl acetate-/hexane as eluant to yield 1.46 g (55.9%) of 4,5-dihydro-5-propyl-4-oxo-4H-thieno[2,3-b]thiophene-6,6-dioxide (**43**) as a pale, viscous oil:
$^1H$ NMR (δ from TMS in $CDCl_3$in ppm): 7.84 (d, 1H, J=5.0 Hz), 7.37 (d, 1H, J=5.0 Hz), 4.23 (dd, 1H, J=7.5 Hz), 2.16 (m, 2H), 1.81 - 1.69 (m, 2H), 1.08 (t, 3H, J=7.5 Hz).

EXAMPLE 9

4,5-Dihydro-4-hydroxyl-5-propyl-4H-thieno[2,3-b]thiophene-6,6-dioxide (**44**):

To a mixture of sodium borohydride (0.042 g, 0.79 mmol) in ethanol (2 ml) was added 4,5-dihydro-5-propyl-4-oxo-4H-thieno[2,3-b]thiophene-6,6-dioxide (**43**) (0.114 g, 0.495 mmol) in dry tetrahydrofuran (4 ml) and the resulting mixture was allowed to stir at room temperature for 5 hours. The reaction was quenched with water

and sat. $NaHCO_3$ and the aqueous was extracted with ethyl acetate (4 x 20 ml). The combined extracts were dried over $MgSO_4$ and concentrated. The product was purified by flash chromatography with silica gel and 20% ethyl acetate/hexane as eluant to yield 0.050 g (43%) of the 4,5-dihydro-4-hydroxyl-5-propyl-4H-thieno[2,3-b]-thiophene-6,6-dioxide (α isomer) (**44a**):
$^1H$ NMR: (δ from TMS in $CDCl_3$ in ppm): 7.68 (d, 1H, J=4.9 Hz), 7.07 (d, 1H, J=4.9 Hz), 4.90 (dd, 1H, J=8.2 Hz), 3.76 - 3.69 (m, 1H), 3.46 (d, 1H, J=8.2 Hz), 2.14 - 2.05 (m, 1H), 1.99 - 1.87 (m, 1H), 1.71 - 1.58 (m, 2H), 1.02 (t, 3H, J=7.2 Hz); and 0.050 g (43%) of 4,5-dihydro-4-hydroxyl-5-propyl-4H-thieno[2,3-b]thiophene-6,6-dioxide (β isomer) (**44b**): $^1H$ NMR: (δ from TMS in $CDCl_3$ in ppm): 7.71 (d, 1H, J=4.9 Hz), 7.10 (d, 1H, J=4.9 Hz), 5.10 (dd, 1H, J=10.0 Hz), 3.91 - 3.84 (m, 1H), 2.51 (d, 1H, J=10.0 Hz), 2.08 - 1.94 (m, 2H), 1.68 - 1.61 (m, 2H), 1.05 (t, 3H, J=7.3 Hz).

EXAMPLE 10

4,5-Dihydro-4-hydroxy-5-propyl-4H-thieno[2,3-b]thiophene-2-sulfonamide-6,6-dioxide (α and β isomer) (**45**):

To a solution of 4,5-dihydro-4-hydroxyl-5-propyl-4H-thieno[2,3-b]thiophene-6,6-dioxide (α and β isomers) (**44a**) and (**44b**) (0.469 g, 2.02 mmol) in anhydrous tetrahydrofuran (20 ml) at -80°C (diethyl ether, dry ice) was added n-butyllithium (1.6 ml, 4.0 mmol) as a 2.5 M solution in hexanes. The resulting orange solution was stirred at -80°C for 1.5 hour. Additional n-butyllithium (0.20 ml, 0.50 mmol) was introduced and the reaction was allowed to continue for 1 hour. This solution was added to a flask which contained anhydrous $SO_2$ in anhydrous tetrahydrofuran (5 ml) at -78°C. The resulting yellow solution was allowed to stir at -78°C for 15 minutes and then was warmed to room temperature for 0.75 hour. The solvent was removed by evaporation in vacuo to yield an orange, viscous oil. This residue was dissolved in $H_2O$ (10 ml) and THF (2 ml) and cooled in an ice bath. To this was added hydroxylamine-O-sulfonic acid (1.24 g, 11.0 mmol) and sodium acetate (1.45 g, 10.7 mmol) and the resulting solution was allowed to stir with warming to room temperature for 1.5 hour. The reaction mixture was diluted with water (pH=4-5) and extracted with ethyl acetate (3 x 30 ml). The aqueous phase was made neutral with $NaHCO_3$ and was extracted with ethyl acetate (3 x 30 ml). The aqueous phase was then brought up to pH=8 and extracted with ethyl acetate (2 x 30 ml). The organic extracts were combined, dried over $MgSO_4$ and concentrated. The product was purified by flash chromatography with silica gel and 30% - 60% ethyl acetate/hexane as eluant to yield 0.084 g (13%) of 4,5-dihydro-4-hydroxyl-5-propyl-4H-thieno[2,3-b]thiophene-2-sulfonamide-6,6-dioxide (α isomer) (**45a**):
$^1H$ NMR: (δ from TMS in $CDCl_3$ in ppm): 8.12 (s, 2H), 7.57 (s, 1H), 6.55 (bs, 1H), 4.94 (d, 1H, J=5.37 Hz), 3.73 (q, 1H, J=8.55 Hz), 2.02 - 1.90 (m, 2H), 1.63 - 1.55 (m, 2H), 0.99 (t, 3H, J=7.38 Hz); and 0.072 g (11%) of 4,5-dihydro-4-hydroxyl-5-propyl-4H-thieno[2,3-b]thiophene-2-sulfonamide-6,6-dioxide (β isomer) (**45b**):
$^1H$ NMR: (δ from TMS in $CDCl_3$ in ppm): 8.09 (s, 2H), 7.58 (s, 1H), 6.18 (bs, 1H), 5.16 (d, 1H, J=5.86 Hz), 4.04 (q, 1H, J=6.0 Hz), 1.87 - 1.79 (m, 2H), 1.56 - 1.48 (m, 2H), 0.98 (t, 3H, J=7.2 Hz); and 0.151 g (24%) of 4,5-dihydro-4-hydroxyl-5-propyl-4H-thieno[2,3-b]thiophene-2-sulfonamide-6,6-dioxide (a and b isomers) (**45α, 45β**) for a total yield of 48%.

EXAMPLE 11

4,5-Dihydro-4-azido-5-propyl-4H-thieno[2,3-b]thiophene-2-sulfonamide-6,6-dioxide (α isomer) (**47α**):

To a solution of 4,5-dihydro-4-hydroxyl-5-propyl-4H-thieno[2,3-b]thiophene-2-sulfonamide-6,6-dioxide (β isomer) (**45β**) (3.15 g, 10.1 mmol) in pyridine (100 ml) at room temperature was added, in portions, methanesulfonylchloride (1.3 ml, 1.9 g, 17 mmol) and the mixture (the alcohol was not soluble) was allowed to stir overnight, ca. 19 hours. The product mixture was diluted with ethyl acetate (200 ml) and washed with 1N HCl (3 x 50 ml) and sat. $NaHCO_3$ (50 ml). The organic layer was dried over $MgSO_4$ and concentrated to yield 4,5-dihydro-4-methanesulfonyloxy-5-propyl-4H-thieno [2,3-b]thiophene-2-sulfonamide-6,6-dioxide (β isomer) (**46β**) a yellow oil. The residue was dissolved in N,N'-dimethylformamide (80 ml) and to this was added at room temperature sodium azide (1.4 g, 22 mmol) and the resulting mixture was stirred for 2.5 hours. The mixture was diluted with ethyl acetate and aqueous sat. $NaHCO_3$ and the layers were separated. The aqueous was extracted with ethyl acetate (3 x 100 ml) and the combined extracts were dried over $MgSO_4$ and concentrated. The product was purified by flash chromatography with silica gel (0.040 - 0.063 mm) and 20% - 50% ethyl acetate/hexane as eluant to yield 1.8 g (49%, 85% a) of 4,5-dihydro-4-azido-5-propyl-4H-thieno[2,3-b]thiophene-2-sulfonamide-6,6-dioxide (α isomer) (**47α**):
$^1H$ NMR: (δ from TMS in DMSO in ppm): 8.15 (s, 2H), 7.70 (s, 1H), 5.35 (d, 1H, J=5.6 Hz), 4.08 - 4.01 (m, 1H), 2.05 - 1.87 (m, 1H), 1.84 - 1.80 (m, 1H), 1.60 - 1.52 (m, 2H), 0.98 (t, 3H, J=7.2 Hz).

EXAMPLE 12

4,5-Dihydro-4-azido-5-propyl-4H-thieno[2,3-b]thiophene-2-sulfonamide-6,6-dioxide (β isomer) (**47β**):

To a solution of 4,5-dihydro-4-hydroxyl-5-propyl-4H-thieno[2,3-b]thiophene-2-sulfonamide-6,6-dioxide (α isomer) (**45α**) (3.36 g, 10.8 mmol) in pyridine (100 ml) at room temperature was added, in portions, methanesulfonylchloride (1.4 ml, 2.0 g, 18 mmol) and the mixture (the alcohol was not soluble) was allowed to stir overnight, ca. 19 hours. The product mixture was diluted with ethyl acetate (200 ml) and washed with 1N HCl (3 x 50 ml) and sat. $NaHCO_3$ (50 ml). The organic layer was dried over $MgSO_4$ and concentrated to yield 4,5-dihydro-4-methanesulfonyloxy-5-propyl-4H-thieno[2,3b]thiophene-2-sulfonamide-6,6-dioxide (α isomer) (**46α**) as a yellow oil. The residue was dissolved in N,N'-dimethylformamide (80 ml) and to this was added at room temperature sodium azide (1.6 g, 24.6 mmol) and the resulting mixture was stirred for 2.5 hours. The mixture was diluted with ethyl acetate and aqueous sat. $NaHCO_3$ and the layers were separated. The aqueous was extracted with ethyl acetate (3 x 100 ml) and the combined extracts were dried over $MgSO_4$ and concentrated. The product was purified by flash chromatography with silica gel (0.040 - 0.063 mm) and 20% - 50% ethyl acetate /hexane as eluant to yield 2.7 g (66%) of 4,5-dihydro-4-azido-5-propyl-4H-thieno[2,3-b]thiophene-2-sulfonamide-6,6-dioxide (β isomer) (**47β**):
$^1$H NMR: (δ from TMS in DMSO in ppm): 8.17 (bs, 2H), 7.82 (s, 1H), 5.45 (d, 1H, J=6.1 Hz), 4.42 - 4.35 (m, 1H), 1.95 - 1.80 (m, 2H), 1.56 - 1.48 (m, 2H), 0.99 (t, 3H, J=7.3 Hz).

EXAMPLE 13

4,5-Dihydro-4-amino-5-propyl-4H-thieno[2,3-b]thiophene-2-sulfonamide-6,6-dioxide(α isomer) (**48α**):

To solid 4,5-dihydro-4-azido-5-propyl-4H-thieno[2,3-b]thiophene-2-sulfonamide-6,6-dioxide (α isomer) (**47α**) (1.23 g, 3.64 mmol) was added 10% Pd/C catalyst (0.28 g, 0.23 mmol) followed by addition of ethyl acetate (14 ml) and methanol (7 ml) and the mixture was purged with $H_2$ and then allowed to stir under a blanket of $H_2$ for 18 hours. Fresh catalyst and $H_2$ was added twice more and the reaction was allowed to stir for 8 hours. The reaction mixture was filtered through Celite and the filtrate was concentrated in vacuo to yield a grey-green solid. The solid was washed with methanol/chloroform mixture to yield 0.90 g (80%) of 4,5-dihydro-4-amino-5-propyl-4H-thieno[2,3-b]-thiophene-2-sulfonamide-6,6-dioxide (α isomer) (**48α**):
mp = 174 - 178°C; $^1$H NMR: (δ from TMS in DMSO in ppm): 8.08 (bs, 2H), 7.62 (s, 1H), 4.01 (d, 1H, J=7.1 Hz), 3.75 - 3.68 (m, 1H), 2.62 (b, 2H), 2.02 - 1.92 (m, 2H), 1.63 - 1.48 (m, 2H), 0.97 (t, 3H, J= 7.3 Hz); Anal. Calc. for $C_9H_{14}N_2S_3O_4$: C, 34.82; H, 4.54; N, 9.03. Found: C, 34.47; H, 4.31; N, 8.78.

EXAMPLE 14

4,5-Dihydro-4-amino-5-propyl-4H-thieno[2,3-b]thiophene-2-sulfonamide-6,6-dioxide (β isomer) (**48β**):

To solid 4,5-dihydro-4-azido-5-propyl-4H-thieno[2,3-b]thiophene-2-sulfonamide-6,6-dioxide (β isomer) (**47β**) (1.97 g, 5.84 mmol) was added 10% Pd/C catalyst (0.32 g, 0.30 mmol) followed by addition of ethyl acetate (20 ml) and methanol (10 ml) and the mixture was purged with $H_2$ and then allowed to stir under a blanket of $H_2$ for 16 hours. Fresh catalyst and $H_2$ was added twice more and the reaction was allowed to stir for 24 hours. The reaction mixture was filtered through Celite and the filtrate was concentrated in vacuo to yield an off-white solid. The solid was washed with methanol/chloroform mixture to yield 1.26 g (70%) of 4,5-dihydro-4-amino-5-propyl-4H-thieno[2,3-b]thiophene-2-sulfonamide-6,6-dioxide (β isomer) (**48β**): mp = 183 - 185°C;
$^1$H NMR: (δ from TMS in DMSO in ppm): 8.08 (bs, 2H), 7.63 (s, 1H), 4.48 (d, 1H, J=6.6 Hz), 4.03 (m, 1H), 2.1 (b, 2H), 1.89 - 1.82 (m, 2H), 1.58 - 1.51 (m, 2H), 0.99 (t, 3H, J=7.3 Hz). Anal. Calc. for $C_9H_{14}N_2S_3O_4$: C, 34.82; H, 4.54; N, 9.03. Found: C, 34.68; H, 4.46; N, 8.89.

EXAMPLE 15

4,5-Dihydro-4-(N-ethyl)-amino-5-propyl-4H-thieno[2,3-b]thiophene-2-sulfonamide-6,6-dioxide (α isomer) (**50α**):

To a solution of 4,5-dihydro-4-amino-5-propyl-4H-thieno[2,3-b]thiophene-2-sulfonamide-6,6-dioxide (β isomer) (48α) (0.275 g, 0.886 mmol) in methylene chloride (8.0 ml) and pyridine (1.6 ml) was added in portions at 0°C , acetic anhydride (0.1 ml, 0.11 g, 1.06 mmol) and the resulting solution was warmed to room temperature

and allowed to stir for 1.5 hours. The reaction mixture was washed with aqueous sat. $NaHCO_3$ (2 x 25 ml) and 1 N HCl (25 ml). The organic layer was diluted with ethyl acetate, dried over $MgSO_4$, and concentrated to yield 4,5-dihydro-4-acetamido-5-propyl-4H-thieno[2,3-b]thiophene-2-sulfonamide-6,6-dioxide (α isomer) (**49**α) as a white solid. To a solution of 4,5-dihydro-4-acetamido-5-propyl-4H-thieno[2,3-b]thiophene-2-sulfonamide-6,6-dioxide (α isomer) (**49**α) (0.212 g, 0.60 mmol) in tetrahydrofuran (12 ml) at room temperature was added borane dimethylsulfide complex (0.22 ml, 3.7 mmol) as a 10 M solution of borane and the resulting solution was stirred at room temperature for 15 minutes and then heated to reflux for 2 hours. The reaction was quenched with dilute HCl and extracted with ethyl acetate. The aqueous was made neutral with 10 N NaOH and $NaHCO_3$ and extracted with ethyl acetate (4 x 25 ml) and these organic extracts were combined, dried over $MgSO_4$, and concentrated. To the residue was added ethanolic HCl and the solvent was removed by evaporation in vacuo to yield a solid. The solid was crystallized from ethyl acetate/absolute ethanol to yield 0.066 g (30%) of 4,5-dihydro-4-(N-ethyl)-amino-5-propyl-4H-thieno[2,3-b]thiophene-2-sulfonamide-6,6-dioxide (a isomer) (**50**α) as a fluffy, white solid: mp = 245 - 248°C with decomposition;

$^1H$ NMR: (δ from TMS in DMSO in ppm): 10.05 - 10.35 (b, 2H), 8.23 (s, 2H), 8.03 (s, 1H), 4.97 (bm, 1H), 4.33 (bm, 1H), 3.16 - 3.11 (bm, 1H), 3.05 - 2.95 (bm, 1H), 2.07 - 2.00 (m, 2H), 1.61 - 1.52 (m, 2H), 1.28 (t, 3H, J=6.8 Hz), 0.96 (t, 3H, J=7.3 Hz). Anal. Calc. for $C_{11}H_{18}N_2S_3O_4 \cdot HCl \cdot 0.30\ H_2O$: C, 34.74; H, 5.19; N, 7.37. Found: C, 34.69; H, 4.95; N, 7.38.

EXAMPLE 16

4,5-Dihydro-4-(N-ethyl)-amino-5-propyl-4H-thieno[2,3-b]thiophene-2-sulfonamide-6,6-dioxide (β isomer) (**50**β):

To a solution of 4,5-dihydro-4-amino-5-propyl-4H-thieno[2,3-b]thiophene-2-sulfonamide-6,6-dioxide (ß isomer) (**48**β) (0.398 g, 1.28 mmol) in pyridine (9.0 ml) was added at room temperature, acetyl chloride (0.12 ml, 0.13 g, 1.7 mmol) and the resulting solution was allowed to stir at room temperature for 3 hours. The pyridine was removed by evaporation in vacuo and the residue was dissolved in ethyl acetate. The organic solution was washed with 1 N HCl (3 x 25 ml), dried over $MgSO_4$, and concentrated to yield 4,5-dihydro-4-acetamido-5-propyl-4H-thieno[2,3-b]thiophene-2-sulfonamide-6,6-dioxide (β isomer) (**49**β) as a white solid. To a solution of 4,5-dihydro-4-acetamido-5-propyl-4H-thieno[2,3-b]thiophene-2-sulfonamide-6,6-dioxide (β isomer) (**49**β) (0.43 g, 1.2 mmol) in tetrahydrofuran (25 ml) at room temperature was added borane dimethylsulfide complex (0.45 ml, 4.5 mmol) as a 10 M solution of borane and the resulting solution was stirred at room temperature for 15 minutes and then heated to reflux for 1 hour. The reaction was quenched with dilute HCl and extracted with ethyl acetate. The aqueous was made neutral with 10 N NaOH and $NaHCO_3$ and extracted with ethyl acetate and these organic extracts were combined, dried over $MgSO_4$, and concentrated to yield an off-white solid. The solid was crystallized from ethyl acetate/hexane to yield 0.14 g (35%) of 4,5-dihydro-4-(N-ethyl)-amino-5-propyl-4H-thieno [2,3-b]thiophene-2-sulfonamide-6,6-dioxide (β isomer) (**50**β) as off-white crystals: mp = 127 - 130°C;

$^1H$ NMR: (δ from TMS in DMSO in ppm): 8.07 (b, 2H), 7.61 (s, 1H), 4.44 (d, 1H, J=5.9 Hz), 4.11 (m, 1H), 2.60 - 2.49 (m, 1H), 2.40 - 2.35 (m, 1H), 1.86 - 1.79 (m, 2H), 1.56 - 1.43 (m, 2H), 0.97 (t, 3H, J=7.3 Hz). Anal. Calc. for $C_{11}H_{18}N_2S_3O_4$: C, 39.03; H, 5.36; N, 8.28. Found: C, 39.16; H, 5.29; N, 8.29.

EXAMPLE 17

4,5-Dihydro-4-(N-propyl)-amino-5-propyl-4H-thieno[2,3-b]thiophene-2-sulfonamide-6,6-dioxide (α isomer) (**52**α):

To a solution of 4,5-dihydro-4-amino-5-propyl-4H-thieno[2,3-b]thiophene-2-sulfonamide-6,6-dioxide (α isomer) (**48**α) (0.303 g, 0.976 mmol) in methylene chloride (3.0 ml) and pyridine (1.2 ml) was added in portions at 0°C , propionyl chloride (0.10 ml, 0.10 g, 1.2 mmol) and the resulting solution was stirred at 0°C for 0.5 hour. The reaction mixture was diluted with aqueous sat. $NaHCO_3$ and extracted with ethyl acetate (3 x 20 ml). The combined extracts were washed with 1 N HCl (2 x 25 ml), dried over $MgSO_4$, and concentrated to yield 0.25 g (70%) of 4,5-dihydro-4-propionamido-5-propyl-4H-thieno [2,3-b]thiophene-2-sulfonamide-6,6-dioxide (α isomer) (**51**α) as an off-white solid. The bis amide was also obtained which was hydrolyzed to the desired product by heating in 1 N HCl and methanol to provide 0.051 g (14%) of the desired amide. To a solution of 4,5-dihydro-4-propionamido-5-propyl-4H-thieno[2,3-b]thiophene-2-sulfonamide-6,6-dioxide (a isomer) (**51**α) (0.259 g, 0.707 mmol) in tetrahydrofuran (15 ml) at room temperature was added borane dimethylsulfide complex (0.26 ml, 2.6 mmol) as a 10 M solution of borane and the resulting solution was stirred at room temperature for 15 minutes and then heated to reflux for 2 hours. The reaction was cooled to room temperature, quenched

with ice and dilute HCl and extracted with ethyl acetate (2 x 35 ml). The aqueous was made neutral with 10 N NaOH and $NaHCO_3$ and extracted with ethyl acetate (4 x 30 ml) and these organic extracts were combined, dried over $MgSO_4$, and concentrated. To the residue was added absolute ethanol and ethanolic HCl and the excess solvent and HCl was removed by evaporation in vacuo to yield a white solid. The solid was crystallized from absolute ethanol/hexane to yield 0.045 g (18%) of 4,5-dihydro-4-(N-propyl)-amino-5-propyl-4H-thieno[2,3-b]thiophene-2-sulfonamide-6,6-dioxide (α isomer) (**52**α) as a fluffy, white crystalline solid:
mp = 138 - 140°C; $^1$H NMR: (δ from TMS in DMSO in ppm): 10.0 - 10.2 (b, 2H), 8.25 (s, 2H), 8.04 (s, 1H), 4.99 (bm, 1H), 4.36 (bm, 1H), 3.10 - 3.06 (bm, 1H), 2.90 - 2.88 (bm, 1H), 2.10 - 2.02 (m, 2H), 1.80 - 1.55 (m, 4H), 0.98 (m, 6H). Anal. Calc. for $C_{12}H_{20}N_2S_2O_4 \cdot HCl \cdot 0.60\ CH_3CH_2OH$: C, 38.05; H, 5.95; N, 6.72. Found: C, 38.05; H, 5.90; N, 6.55.

EXAMPLE 18

4,5-Dihydro-4-(N-propyl)-amino-5-propyl-4H-thieno[2,3-b]thiophene-2-sulfonamide-6,6-dioxide (β isomer ) (**52**β):

To a solution of 4,5-dihydro-4-amino-5-propyl-4H-thieno[2,3-b]thiophene-2-sulfonamide-6,6-dioxide (β isomer) (**48**β) (0.341 g, 1.10 mmol) in pyridine (8.0 ml) was added in portions at room temperature, propionyl chloride (0.16 ml, 0.17 g, 1.8 mmol) and the resulting solution was allowed to stir at room temperature for 3 hours. The pyridine was removed by evaporation in vacuo and the residue was diluted with aqueous sat. $NaHCO_3$ and extracted with ethyl acetate (3 x 25 ml). The combined extracts were washed with 1 N HCl (2 x 25 ml), dried over $MgSO_4$, and concentrated to yield 0.41 g (>99%) of 4,5-dihydro-4-propionamido-5-propyl-4H-thieno[2,3-b]thiophene-2-sulfonamide-6,6-dioxide (β isomer) (**51**β) as an off-white solid. To a solution of the β propionamide (**51**β) (0.35 g, 0.96 mmol) in tetrahydrofuran (20 ml) at room temperature was added borane dimethylsulfide complex (0.35 ml, 3.5 mmol) as a 10 M solution of borane and the resulting solution was stirred at room temperature for 20 minutes and then heated to reflux for 1.5 hours. The reaction was cooled to room temperature, quenched with ice and dilute HCl and extracted with ethyl acetate (50 ml). The aqueous was made neutral with 10 N NaOH and $NaHCO_3$ and extracted with ethyl acetate (6 x 30 ml). These organic extracts were combined, dried over $MgSO_4$, and concentrated. The residue was dissolved in absolute ethanol and ethanolic HCl was added. The solvent and excess HCl were removed by evaporation in vacuo and the product was allowed to crystallize from absolute ethanol/hexane to yield 0.15 g (46%) of 4,5-dihydro-4-(N-propyl)-amino-5-propyl-4H-thieno[2,3-b] thiophene-2-sulfonamide-6,6-dioxide (β isomer) (**52**β): mp = 148 - 155°C with decomposition;
$^1$H NMR: (δ from TMS in DMSO in ppm): 9.35 - 9.45 (bs, 1H), 8.22 (b, 2H), 8.01 (s, 1H), 5.16 (bm, 1H), 4.56 (bm, 1H), 2.82 - 2.76 (m, 1H), 2.55 - 2.48 (m, 1H), 2.31 - 2.25 (bm, 1H), 2.00 - 1.92 (bm, 1H), 1.76 - 1.49 (m, 4H), 1.00 (t, 3H, J=7.3 Hz), 0.88 (t, 3H, J=7.3 Hz). Anal. Calc. for $C_{12}H_{20}N_2S_3O_4 \cdot HCl \cdot CH_3CH_2OH \cdot 0.5\ H_2O$: C, 37.87; H, 6.36; N, 6.31. Found: C, 37.97; H, 6.01; N, 6.31.

EXAMPLE 19

4,5-Dihydro-4-oxo-5-[2-(2-methoxy)ethoxyethyl]-4H-thieno[2,3-b]thiophene-6,6-dioxide

Step A: Preparation of Diethyl 2-(3-bromo-2-thienylthio)malonate (**53**):

To a solution of 2,3-dibromothiophene (5.0 g, 20.7 mmol) in diethyl ether (80 ml) at -78°C was added n-butyllithium (9.0 ml, 22.5 mmol) as a 2.5 M solution in hexanes. The reaction was allowed to stir 20 minutes. To this solution was added powdered sulfur (0.98 g, 30.6 mmol) and the reaction was allowed to stir at -78°C for 15 minutes, warmed to -30C over 15 minutes, and then cooled again to -78°C. Diethyl bromomalonate (4.0 ml, 5.6 g, 23.5 mmol) was added and the resulting mixture was allowed to stir at -78°C for 1 hour and then warmed to room temperature for 4 hours. The reaction was quenched with water and aqueous sat. $NaHCO_3$ and the aqueous was extracted with ethyl acetate (3 x 100 ml). The combined extracts were dried over $MgSO_4$. The solvent was removed by evaporation in vacuo. The product was purified by flash chromatography with silica gel and 1.54 - 6% ethyl acetate/hexane as eluant to yield 4.63 g (63%) of diethyl 2-(3-bromo-2-thienylthio)malonate (53) as a yellow oil:
$^1$H NMR (δ from TMS in $CDCl_3$ in ppm): 7.43 (d, 1H, J=5.4 Hz), 7.05 (d, 1H, J=5.4 Hz), 4.51 (s, 1H,), 4.26 - 4.20 (m, 4H), 1.26 (t, 6H, J=7.1 Hz).

Step B: Preparation of Diethyl 2-(3-bromo-2-thienylthio)-2-[2-(2-methoxy)ethoxyethyl]malonate (**54**):

To a stirring suspension of sodium hydride (0.57 g, 14 mmol, 60% NaH in mineral oil) in N,N′-dimethylformamide (20 ml) at 0°C was added, over 30 minutes, diethyl 2-(3-bromo-2-thienylthio)-malonate (**53**) (3.6 g, 10.2 mmol) as a solution in N,N′-dimethylformamide (15 ml) and the resulting solution was stirred at 0°C for 10 minutes. 2-(2-methoxy)ethoxyethyl bromide (1.8 ml, 2.4 g, 13 mmol) was added and the resulting solution was warmed to room temperature over 0.5 hour and then heated to 85°C for 6 hours. Additional bromide (0.60 ml, 0.81 g, 4.4 mmol) was introduced and the reaction was allowed to stir at 80°C for 15 hours. Aqueous sat. $NaHCO_3$ was added and the solution was extracted with ethyl acetate (4 x 50 ml). The combined extracts were washed with 2 N HCl (50 ml), sat. brine (50 ml), and dried over $MgSO_4$. The product was purified by flash chromatography with silica gel (0.040 mm - 0.063 mm) and 7%- 30% ethyl acetate/hexane as eluant to yield 2.80 g (60%) of diethyl 2-(3-bromo-2-thienylthio)-2-[2-(2-methoxy)-ethoxy-ethyl]-malonate (**54**) as an opaque yellow oil: $^1H$ NMR (δ from TMS in $CDCl_3$ in ppm): 7.46 (d, 1H, J=5.6 Hz), 7.04 (d, 1H, J=5.6 Hz), 4.23 (q, 4H, J=7.1 Hz), 3.85 (t, 2H, J=6.0 Hz), 3.58 - 3.55 (m, 2H), 3.52 - 3.49 (m, 2H), 3.37 (s, 3H), 2.37 (t, 2H, J=6.0 Hz), 1.27 (t, 6H, J=7.1 Hz).

Step C: Preparation of 2-(3-Bromo-2-thienylthio)4-(2-methoxy)-ethoxy butylic acid (**55**):

To a solution of diethyl 2-(3-bromo-2-thienylthio)-2-[2-(2-methoxy)-ethoxy-ethyl]-malonate (**54**) (2.80 g, 6.15 mmol) in absolute ethanol (60 ml) was added sodium hydroxide (2.0 ml, 20 mmol) as a 10 N aqueous solution and the resulting mixture was heated to reflux for 5 hours. The opaque mixture was allowed to cool and made acidic with aqueous 2N HCl and extracted with ethyl acetate (4 x 30 ml). The combined extracts were washed with sat. brine (50 ml), dried over $MgSO_4$, and concentrated to yield 1.93 g (88%) of 2-(3-bromo-2-thienylthio)-4-(2-methoxy)ethoxy butyric acid (55) as a yellow oil:
$^1H$ NMR (δ from TMS in $CDCl_3$ in ppm): 9.70 (bs, 1H), 7.42 (d, 1H, J=5.6 Hz), 7.04 (d, 1H, J=5.6 Hz), 3.78 - 3.70 (m, 1H), 3.62 - 3.52 (m, 6H), 3.39 (s, 3H), 2.28 - 1.98 (m, 2H).

Step D: Preparation of 2-(3-Bromo-2-thienylthio)-4-(2-methoxy)ethoxy-N-methyl-N-methoxybutyramide (**56**):

To a solution of 2-(3-bromo-2-thienylthio)-4-(2-methoxy)-ethoxy butyric acid (**55**) (1.93 g, 5.43 mmol) in N,N′-dimethylformamide (8.0 ml) was added, portionwise at room temperature, N, N′-carbonyldiimidazole (2.1 g, 13 mmol) and the resulting solution was stirred for 10 minutes or until $CO_2$ evolution could no longer be observed. To this solution was added N,O-dimethylhydroxylamine hydrochloride (1.2 g, 12.3 mmol) in N,N′-dimethylformamide (8.0 ml). The resulting mixture was stirred for 4 hours and then the DMF was removed by evaporation in vacuo. The residue was diluted with water and $NaHCO_3$ solution and extracted with ethyl acetate (3 x 30 ml). The combined extracts were washed with 1 N HCl (2 x 50 ml), then with sat. brine (2 x 50 ml) and dried over $MgSO_4$/$NaHCO_3$ and concentrated to yield 1.87 g (86%) of 2-(3-bromo-2-thienylthio)-4-(2-methoxy)-ethoxy-N-methyl-N-methoxy-butyramide (**56**) as a yellow oil:
$^1H$ NMR (δ from TMS in $CDCl_3$ in ppm): 7.39 (d, 1H, J=5.4 Hz), 7.03 (d, 1H, J=5.4 Hz), 4.25 (m, 1H), 3.70 - 3.67 (m, 1H), 3.69 (s, 3H), 3.60 - 3.45 (m, 5H), 3.61 (s, 3H), 3.20 (s, 3H).

Step E: Preparation of 4,5-Dihydro-4-oxo-5-[2-(2-methoxy)ethoxyethyl]-4H-thieno[2,3-b]thiophene-6,6-dioxide(**57**):

Prepared in the same fashion as 4,5-dihydro-5-propyl-4-oxo-4H-thieno[2,3-b]thiophene-6,6-dioxide (43) to obtain 4,5-dihydro-4-oxo-5-[2-(2-methoxy)ethoxy-ethyl]-4H-thieno[2,3-b]thiophene-6,6-dioxide (57) in 55% for the 2 steps: mp: 55 - 60°C;
$^1H$ NMR (δ from TMS in $CDCl_3$ in ppm): 7.78 (d, 1H, J=5.0 Hz), 7.34 (d, 1H, J=5.0 Hz), 4.54 (q, 1H, J=4.6 Hz), 3.86 - 3.75 (m, 2H), 3.69 - 3.65 (m, 2H), 3.58 - 3.54 (m, 2H), 3.39 (s, 3H), 2.52 - 2.35 (m, 2H).

EXAMPLE 20

4,5-Dihydro-4-hydroxyl-5-[2-(2-methoxy)ethoxyethyl]-4H-thieno[2,3-b]thiophene-6,6-dioxide (α and β isomer (**58**):

To a solution of 4,5-dihydro-4-oxo-5-[2-(-2-methoxy) ethoxyethyl]-4H-thieno[2,3-b]thiophene-6,6-dioxide (**57**) (9.18 g, 31-.6 mmol) in absolute-ethanol (100 ml) and tetrahydrofuran (100 ml) at 0°C was added sodium borohydride (0.75 g, 20 mmol) and the resulting solution was stirred at 0°C for 0.75 hour and warmed to room

temperature for 0.5 hour. The reaction mixture was carefully quenched with ice and dilute HCl and extracted with diethyl ether (3 x 100 ml) and ethyl acetate (3 x 100 ml). The combined organic extracts were dried over $MgSO_4$ and concentrated to afford a pale yellow oil. The product was purified by flash chromatography with silica gel (0.040-0.063 mm) and 40% - 100% ethyl acetate/hexane as eluant to yield 3.04 g (33%) of 4,5-dihydro-4-hydroxyl-5-[2-(2-methoxy)-ethoxyethyl]-4H-thieno[2,3-b]thiophene-6,6-dioxide (α isomer) (**58**α) as a white solid:

$^1$H NMR (δ from TMS in $CDCl_3$ in ppm): 7.66 (d, 1H, J=4.9 Hz), 7.13 (d, 1H, J=4.9 Hz), 5.27 (bs, 1H), 5.08 (d, 1H, J=5.4 Hz), 3.90 - 3.85 (m, 1H), 3.80 - 3.50 (m, 6H), 3.41 (s, 3H), 2.18 - 2.38 (m, 2H); and 5.51 g (60%) of 4,5-dihydro-4-hydroxyl-5-[2-(2-methoxy)-ethoxy-ethyl]-4H-thieno[2,3-b]thiophene-6,6-dioxide (β isomer) (**58**β) as an oily solid:

$^1$H NMR (δ from TMS in $CDCl_3$ in ppm): 8.07 (d, 1H, J=4.9 Hz), 7.17 (d, 1H, J=4.9 Hz), 5.33 (d, 1H, J=5.6 Hz), 3.93 - 4.00 (m, 1H), 3.87 - 3.83 (m, 1H), 3.75 - 3.50 (m, 5H), 3.40 (s, 3H), 2.60 - 2.45 (m, 1H), 2.35 - 2.20 (m, 1H).

## EXAMPLE 21

4,5-Dihydro-4-hydroxyl-5-[2-(2-methoxy)ethoxyethyl]-4H-thieno[2,3-b]thiophene-2-sulfonamide-6,6-dioxide (β isomer) (**59**β):

To a solution of 4,5-dihydro-4-hydroxyl-5-[2-(2-methoxy)ethoxyethyl]-4H-thieno[2,3-b]thiophene-6,6-dioxide (β isomer) (**58**β) (1.54 g, 5.26 mmol) in anhydrous tetrahydrofuran (40 ml) at -78°C was added n-butyllithium (4.50 ml, 11.3 mmol) as a 2.5 M solution in hexanes and the resulting solution was stirred for 20 minutes. To this was added anhydrous $SO_2$ gas by slowing blowing the gas, in portions, over the surface of the liquid until the pH 4. The reaction mixture was allowed to stir at 78°C for 1 hour and them warmed to room temperature. The solvent and excess $SO_2$ gas was removed by evaporation in vacuo and the residue was dissolved in water (15 ml) and tetrahydrofuran (2 ml). Sodium acetate (4.3 g, 32 mmol) and hydroxylamine-O-sulfonic acid (3.0 g, 26 mmol), respectively, were added in two portions and the resulting solution was allowed to stir overnight, ca. 16 hours. The reaction mixture was diluted with aqueous sat. $NaHCO_3$ and extracted with ethyl acetate. The combined extracts were dried over $MgSO_4$/$NaHCO_3$ and concentrated. The product was purified by flash chromatography with silica gel (0.040 - 0.063 mm) and 7% - 10% methanol/chloroform as eluant to yield 3.0 g (47%) of 4,5-dihydro-4-hydroxyl-5-[2-(2-methoxy)-ethoxy-ethyl]-4H-thieno[2,3b]thiophene-2-sulfonamide-6,6-dioxide (β isomer) (**59**β) as an oily solid:

$^1$H NMR (δ from TMS in DMSO in ppm): 8.10 (s, 2H), 7.60 (s, 1H), 6.23 (d, 1H, J=5.9 Hz), 5.20 (m, 1H), 4.08 (m 1H), 3.61 (t, 2H,-J=6.4 Hz), 3.,56 - 3.53 (m,, 2H),-3.47 - 3.44 (m, 2H), 3.25 (s, 3H), 2.09 (m, 2H).

## EXAMPLE 22

4,5-Dihydro-4-azido-5-[2-(2-methoxy)ethoxyethyl]-4H-thieno[2,3-b]thiophene-2-sulfonamide-6,6-dioxide (β isomer) (**61**β):

To a solution of 4,5-dihydro-4-hydroxyl-5-[2-(2-methoxy)-ethoxy-ethyl]-4H-thieno[2,3b]thiophene-2-sulfonamide-6,6-dioxide (β isomer) (**59**β) (2.67g, 7.19 mmol) in pyridine (35 ml) at -30°C was added methanesulfonylchloride (0.70 ml, 1.0 g, 9.0 mmol) and the solution was allowed to stir with slow warming to room temperature for 3.5 hours. The product mixture was diluted with a small amount of water and 1N HCl and the pyridine was removed by evaporation in vacuo. The residue was diluted with aqueous HCl and some sat. brine and extracted with ethyl acetate (3 x 75 ml). The combined extracts were dried over $MgSO_4$ and concentrated to yield 4,5-dihydro-4-methanesulfonyloxy-5-[2-(2-methoxy)ethoxy-ethyl]-4H-thieno[2,3-b]thiophene-2-sulfonamide-6,6-dioxide (β isomer) (**60**β) as a yellow oil. The residue was dissolved in N,N′-dimethylformamide (35 ml) and to this was added sodium azide (0.91 g, 14 mmol) and the resulting mixture was stirred at room temperature for 16 hours and then heated to 50°C for 3 hours. The mixture was diluted with aqueous sat. $NaHCO_3$ and the aqueous was extracted with ethyl acetate (3 x 75 ml) and the combined extracts were dried over $MgSO_4$ and concentrated. The product was purified by flash chromatography with silica gel (0.040 - 0.063 mm) and 2% - 5% methanol/chloroform as eluant to yield 1.85 g (65%) of 4,5-dihydro-4-azido-5-[2-(2-methoxy)-ethoxyethyl]-4H-thieno[2,3-b]thiophene-2-sulfonamide-6,6-dioxide (β isomer) (**61**β):

$^1$H NMR (δ from TMS in DMSO in ppm): 8.15 (s, 2H), 7.73 (s, 1H), 5.43 (d, 1H, J=5.1 Hz), 4.05 - 4.0 (m, 1H), 3.66 (t, 2H, J=6.3 Hz), 3.57 - 3.53 (m, 2H), 3.50 - 3.45 (m, 2H), 3.26 (s, 3H), 2.34 - 2.23 (m, 1H), 2.15 - 2.05 (m, 1H).

EXAMPLE 23

4,5-Dihydro-4-amino-5-[2-(2-methoxy)ethoxyethyl]-4H-thieno[2,3-b]thiophene-2-sulfonamide-6,6-dioxide (β isomer) (**62**β):

To solid 4,5-dihydro-4-azido-5-[2-(2-methoxy)-ethoxy-ethyl]-4H-thieno[2,3-b]thiophene-2-sulfonamide-6,6-dioxide (β isomer) (**61**β) (1.48 g, 3.73 mmol) was added 10% Pd/C catalyst (0.20 g, 0.19 mmol Pd) followed by addition of ethyl acetate (30 ml) and methanol (10 ml) and the mixture was purged with $N_2$ by bubbling $N_2$ through the reaction mixture. The reaction mixture was then purged with $H_2$ and allowed to stir under a blanket of $H_2$ (with frequent purging of $H_2$) for 5 hours. The reaction mixture was filtered through a pad of Celite and the filtrate was concentrated in vacuo to yield a grey-green solid. The solid was purified by flash chromatography with silica gel (0.040 - 0.063 mm) and 3% - 12% methanol/chloroform to yield 1.34 g (97%) of 4,5-dihydro-4-amino-5-[2-(2-methoxy)-ethoxy-ethyl]-4H-thieno[2,3-b]thiophene-2-sulfonamide-6,6-dioxide (β isomer) (**62ß**) as an off-white foam:
$^1$H NMR (δ from TMS in DMSO in ppm): 8.02 (bs, 2H), 7.57 (s, 1H), 4.04 (d, 1H, J=7.1 Hz), 3.74 - 3.70 (m, 1H), 3.65 - 3.48 (m, 4H), 3.43 - 3.40 (m, 2H), 3.20 (s, 3H), 2.55 (bs, 2H), 2.28 - 2.05 (m, 2H).

EXAMPLE 24

4,5-Dihydro-4-(N-ethyl)-amino-5-[2-(2-methoxy)ethoxyethyl]-4H-thieno[2,3-b]thiophene-2-sulfonamide-6,6-dioxide (β isomer) (**64**β):

The title compound was prepared in a manner similar to 4,5-dihydro-4-(N-ethyl)-amino-5-propyl-4H-thieno[2,3-b]thiophene-2-sulfonamide-6,6-dioxide (α isomer) (**50**α) to afford 0.63 g (63%) of the HCl salt of 4,5-dihydro-4-(N-ethyl)-amino-5-[2-(2-methoxy)-ethoxy-ethyl]-4H-thieno[2,3-b]thiophene-2-sulfonamide-6,6-diox ide β isomer) (**64**β):
$^1$H NMR (δ from TMS in DMSO in ppm): 10.20 (bs, 2H), 8.24 (s, 2H), 8.10 (s, 1H), 5.10 - 5.05 (bm, 1H), 4.39 - 4.35 (bm, 1H), 3.65 (t, 2H, J=6.4 Hz), 3.58 - 3.50 (m, 2H), 3.48 - 3.45 (m, 2H), 3.26 (s, 3H), 3.15 (bm, 1H), 2.97 (bm, 1H), 2.40 - 2.26 (m, 2H), 1.05 (t, 3H, J=7.1 Hz).

The trans racemate is resolved by crystallization as the + or - di-p-toluoyl tartaric acid salt followed by treatment with HCl to yield either the + or - enantiomer as the HCl salt. The cis isomer is resolved similarly.

EXAMPLE 25

4,5-Dihydro-4-oxo-5-(2-methoxy)ethyl-4H-thieno[2,3-b]thiophene-6,6-dioxide

Step A: Preparation of Ethyl 2-(3-bromo-2-thienylthio)acetate (**65**):

The title compound was prepared in a manner similar to diethyl 2-(3-bromo-2-thienylthio)-malonate (**53**) to afford 120 g (80%) of ethyl 2-(3-bromo-2-thienylthio)-acetate (**65**) as an orange oil:
$^1$H NMR (δ from TMS in $CDCl_3$ in ppm): 7.38 (d, 1H, J=5.6 Hz), 7.03 (d, 1H, J=5.6 Hz), 4.16 (q, 2H, J=7.1 Hz), 3.52 (s, 2H), 1.25 (t, 3H, J=7.1 Hz).

Step B: Preparation of Ethyl 2-(3-bromo-2-thienylthio)-4-methoxybutyrate (**66**):

The title compound was prepared in the same fashion as diethyl 2-(3-bromo-2-thienylthio)-2-[2-(2-methoxy)-ethoxyethyl]-malonate (54) to afford 55.4 g (38%) of ethyl 2-(3-bromo-2-thienylthio)-4-methoxy-butyrate (**66**) as an orange oil:
$^1$H NMR (δ from TMS in $CDCl_3$ in ppm): 7.40 (d, 1H, J=5.6 Hz), 7.03 (d, 1H, J=5.6 Hz), 4.14 (q, 2H, J=7.1 Hz), 3.74 (t, 1H, J=7.8 Hz), 3.64 - 3.56 (m, 1H), 3.50 - 3.42 (m, 1H), 3.39 - 3.28 (m, 1H), 3.24 (s, 3H), 1.96 - 2.22 (m, 2H), 1.23 (t, 3H, J=7.1 Hz).

Step C: Preparation of 2-(3-Bromo-2-thienylthio)-4-methoxybutyric acid (**67**):

The title compound was prepared in a manner similar to 2-(3-bromo-2-thienylthio)-4-(2-methoxy)ethoxy butyric acid (**55**) to afford 70.9 g (96%) of 2-(3-bromo-2-thienylthio)-4-methoxy-butyric acid (**67**) as an orange oil:
$^1$H NMR (δ from TMS in $CDCl_3$ in ppm): 10.85 (bs, 1H), 7.41 (d, 1H, J=5.6 Hz), 7.05 (d, 1H, J=5.6 Hz), 3.74 (t,

1H, J=7.3 Hz), 3.66 - 3.60 (m, 1H), 3.55 - 3.48 (m, 1H), 3.34 (s, 3H), 2.20 - 2.00 (m, 2H).

Step D: Preparation of 2-(3-Bromo-2-thienylthio)-4-methoxy-N-methyl-N-methoxybutyramide (**68**):

The title compound was prepared in a manner similar to 2-(3-bromo-2-thienylthio)-4-(2-methoxy)ethoxy-N-methyl-N-methoxybutyramide (**56**) to afford 71.6 g (92%) of 2-(3-bromo-2-thienylthio)-4-methoxy-N-methyl-N-methoxy-butyramide (**68**) as a yellow oil:
$^1$H NMR (δ from TMS in $CDCl_3$ in ppm): 7.39 (d, 1H, J=5.6 Hz), 7.03 (d, 1H, J=5.6 Hz), 4.26 (m, 1H), 3.67 (s, 3H), 3.60 - 3.54 (m, 1H), 3.42 - 3.34 (m, 1H), 3.30 (s, 3H), 3.19 (s, 3H), 2.22 - 2.00 (m, 2H).

Step E: Preparation of 4,5-Dihydro-4-oxo-5-(2-methoxy)-ethyl-4H-thieno[2,3-b]thiophene-6,6-dioxide (**69**):

The title compound was prepared in the same fashion as 4,5-dihydro-5-propyl-4-oxo-4H-thieno[2,3-b]thiophene-6,6-dioxide (**43**) to afford 28 g (61%) of 4,5-dihydro-4-oxo-5-(2-methoxy)-ethyl-4H-thieno[2,3-b]thiophene-6,6-dioxide (**69**) as an off-white solid: mp=103 - 107°C; $^1$H NMR (δ from TMS in $CDCl_3$ in ppm): 7.78 (d, 1H, J=5.1 Hz), 7.34 (d, 1H, J=5.1 Hz), 4.50 - 4.46 (m, 1H), 3.73 - 3.63 (m, 2H), 3.40 (s, 3H), 2.48 - 2.35 (m, 2H); Anal. Calc. for $C_9H_{10}S_2O_4$: C, 43.89; H, 4.09. Found: C, 43.81; H, 4.11.

EXAMPLE 26

4,5-Dihydro-4-hydroxyl-5-(2-methoxy)ethyl-4H-thieno[2,3-b]thiophene-6,6-dioxide (α and β isomers) (**70**):

The title compound was prepared in a manner similar to 4,5-dihydro-4-hydroxyl-5-[2-(2-methoxy)ethoxy-ethyl]-4H-thieno[2,3-b]thiophene-6,6-dioxide (α and β isomers) (**58**) to afford 21.4 g (98%) of 4,5-dihydro-4-hydroxyl-5-(2-methoxy)-ethyl-4H-thieno[2,3-b]thiophene-6,6-dioxide (α and β isomers) (**70a**)and **70b**) as a 1:1 mixture of isomers. (α isomer): mp = 99.5 - 102.5°C;
$^1$H NMR (δ from TMS in $CDCl_3$ in ppm): 7.70 (d, 1H, J=4.9 Hz), 7.14 (d, 1H, J=4.9 Hz), 5.01 (dd, 1H, J=2.7 Hz, 6.2 Hz), 4.65 (d, 1H, J=2.7 Hz), 3.82 - 3.65 (m, 3H), 3.49 (s, 3H), 2.40 - 2.20 (m, 2H). (β isomer): mp = 78 -80°C;
$^1$H NMR (δ from TMS in $CDCl_3$ in ppm): 7.70 (d, 1H, J=4.9 Hz), 7.11 (d, 1H, J=4.9 Hz), 5.30 (d, 1H, J=6.1 Hz), 4.01 - 3.95 (m, 1H), 3.90 (bs, 1H), 3.78 - 3.73 (m, 1H), 3.61 - 3.53 (m, 1H), 3.39 (s, 3H), 2.52 - 2.23 (m, 2H).

EXAMPLE 27

4,5-Dihydro-4-hydroxyl-5-(2-methoxy)ethyl-4H-thieno[2,3-b]thiophene-2-sulfonamide-6,6-dioxide (α and β isomer) (**71**):

The title compound was prepared in a manner similar to 4,5-dihydro-4-hydroxyl-5-[2-(2-methoxy)ethoxy-ethyl]-4H-thieno[2,3-b]thiophene-2-sulfonamide-6,6-dioxide (β isomer) (**59b**) to afford 0.72 g (55%) of 4,5-dihydro-4-hydroxyl-5-(2-methoxy)-ethyl-4H-thieno[2,3-b]thiophene-2-sulfonamide-6,6-dioxide (α and β isomers) (**71a**)and **71b**) as a mixture of isomers: mp=128 - 134°C;
$^1$H NMR (δ from TMS in DMSO in ppm) of major isomer: 8.10 (s, 2H), 7.55 (s, 1H), 6.55 (d, 1H, J=7.1 Hz), 5.00 (t, 1H, J=7.1 Hz), 3.83 - 3.76 (m, 1H), 3.58 - 3.51 (m, 2H), 3.28 (s, 3H), 2.24 - 2.08 (m, 2H).

Employing the procedures substantially as described in the foregoing examples but employing appropriate agents there are also produced the compounds described in the following table:

## TABLE

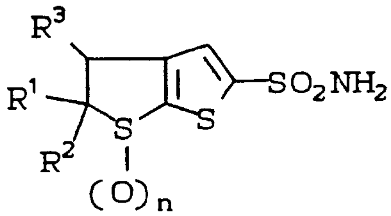

| $R^1$ | $R^2$ | $R^3$ | n |
|---|---|---|---|
| H | $-(CH_2)_2OCH_3$ | $-NHCH_2CH_2CH_3$ | 2 |
| $-CH_3$ | $-CH_2CH_3$ | $-NHCH_2CH_3$ | 2 |
| $-CH_3$ | $-(CH_2)_3OCH_3$ | $-NHCH_2CH_2CH_3$ | 2 |
| H | $-(CH_2)_3OCH_3$ | $-NHCH_2CH_3$ | 2 |
| $n-C_3H_7$ | $-(CH_2)_3OCH_3$ | $-NHCH_2CH_3$ | |
| H | $-(CH_2)_3OCH_3$ | $-NHCH_2CH_3$ | 2 |
| $-CH_3$ | $-CH_2O(CH_2)_2OCH_3$ | $-NHCH_2CH_3$ | 2 |
| $-CH_3$ | $-(CH_2)_2O(CH_2)_2OCH_3$ | $-NHCH_2CH_3$ | 2 |
| $-CH_3$ | $-CH_2SO_2CH_3$ | $-NHCH_2CH_3$ | 2 |
| $-CH_3$ | $-(CH_2)_2SO_2CH_3$ | $-NHCH_2CH_3$ | 2 |
| H | $-CH_2CN$ | $-NHCH_2CH_3$ | 2 |
| H | $-CH_2CO-NH_2$ | $-NHCH_2CH_3$ | 2 |
| H | $-CH_2CO_2H$ | $-NHCH_2CH_3$ | 2 |
| H | $-(CH_2)_2CN$ | $-NHCH_2CH_3$ | 2 |
| H | $-(CH_2)_2CO-NH_2$ | $-NHCH_2CH_3$ | 2 |
| H | $-(CH_2)_2CO_2H$ | $-NHCH_2CH_3$ | 2 |
| H | $-(CH_2)_3CO_2H$ | $-NHCH_2CH_3$ | 2 |
| H | $-(CH_2)_3CO-NH_2$ | $-NHCH_2CH_3$ | 2 |
| H | $-(CH_2)_2SCH_3$ | $-NHCH_2CH_3$ | 2 |
| H | $-(CH_2)_2SO-CH_3$ | $-NHCH_2CH_3$ | 2 |

| $R^1$ | $R^2$ | $R^3$ | n |
|---|---|---|---|
| H | $-(CH_2)_2SO_2-CH_3$ | $-NHCH_2CH_3$ | 2 |
| H | $-(CH_2)_3O(CH_2)_2-O-CH_3$ | $-NHCH_2CH_3$ | 2 |
| H | $-(CH_2)_2O-(CH_2)_2-OCH_3$ | $-NHCH_2CH_2CH_3$ | 2 |
| H | $-(CH_2)_2C{\equiv}N$ | $-NHCH_2CH_2CH_3$ | 2 |
| H | $-(CH_2)_2CO-NH_2$ | $-NHCH_2CH_2CH_3$ | 2 |
| H | $-(CH_2)_2COOH$ | $-NHCH_2CH_2CH_3$ | 2 |
| H | $-(CH_2)3-SO-CH_3$ | $-NHCH_2CH_3$ | 2 |
| H | $-(CH_2)_3-SO_2CH_3$ | $-NHCH_2CH_3$ | 2 |
| H | $-(CH_2)_2-OH$ | $-NHCH_2CH_3$ | 2 |
| H | $-(CH_2)_2O(CH_2)_2-OH$ | $-NHCH_2CH_3$ | 2 |
| H | $-(CH_2)_2-S-(CH_2)_2-OH$ | $-NHCH_2CH_3$ | 2 |
| H | $-(CH_2)_2-SO-(CH_2)_2-OH$ | $-NHCH_2CH_3$ | 2 |
| H | $-(CH_2)_2-SO_2-(CH_2)_2-OH$ | $-NHCH_2CH_3$ | 2 |
| H | $-(CH_2)_2-N(CH_2)_2-OH$ | $-NHCH_2CH_3$ | 2 |
| H | $-(CH_2)_2CH_3$ | $NH(CH_2)_2-OH$ | 2 |
| H | $-(CH_2)_2CH_3$ | $-NH(CH_2)_3-OCH_3$ | 2 |
| H | $-(CH_2)_2CH_3$ | $-NH(CH_2)_2-SCH_3$ | 2 |
| H | $-(CH_2)_2CH_3$ | $-NH(CH_2)_2-SOCH_3$ | 2 |
| H | $-(CH_2)_2CH_3$ | $-NH(CH_2)_2-SO_2CH_3$ | 2 |
| H | $-(CH_2)_2CH_3$ | $-NHCH_2CH(CH_3)_2$ | 2 |
| H | $-(CH_2)_2O(CH_2)_2-OCH_3$ | $-NHCH_2CH(CH_3)_2$ | 2 |
| H | $-(CH_2)_2O(CH_2)_2-OCH_3$ | $-NHCH_2CH_2-F$ | 2 |
| H | $-(CH_2)_2O(CH_2)_2-OCH_3$ | $-NHCH_2CH_2-OH$ | 2 |
| H | $-CH_2OCH_3$ | $-NHCH_2CH_3$ | 2 |
| H | $-CH_2OCH_2CH=CH_2$ | $-NHCH_2CH_3$ | 2 |
| H | $-(CH_2)_3O(CH_2)_2-OCH_3$ | $-NHCH_2CH_3$ | 2 |

EXAMPLE 28

EYE DROP FORMULATION

| | | |
|---|---|---|
| Novel Compound | 1 mg | 15 mg |
| Monobasic sodium phosphate $2H_2O$ | 9.38 mg | 6.10 mg |
| Dibasic sodium phosphate $.12H_2O$ | 28.48 mg | 16.80 mg |
| Benzalkonium chloride | 0.10 mg | 0.10 mg |
| Water for injection q.s. | 1.0 ml | 1.0 ml |

The novel compound, phosphate buffer salts, and benzalkonium chloride are added to and dissolved in water. The pH of the composition is adjusted to 6.8 and diluted to volume. The composition is rendered sterile by ionizing radiation.

EXAMPLE 29

OPHTHALMIC OINTMENT FORMULATION

| | |
|---|---|
| Novel Compund | 5 mg |
| petrolatum q.s. | 1 gram |

The compound and the petrolatum are aseptically combined.

EXAMPLE 30

OPHTHALMIC INSERT FORMULATION

| | |
|---|---|
| Novel Compound | 1 mg |
| Hydroxypropylcellulose q.s. | 12 mg |

Ophthalmic inserts are manufactured from compression molded films which are prepared on a Carver Press by subjecting the powdered mixture of the above ingredients to a compressional force of 12,000 lbs. (gauge) at 300°F for one to four minutes. The film is cooled under pressure by having cold water circulate in the platen. Ophthalmic inserts are then individually cut from the film with a rod-shaped punch. Each insert is placed into a vial, which is then placed in a humidity cabinet (88% R.H. at 30°C) for two to four days. After removal from the humidity cabinet, the vials are stoppered and then autocalved at 250°F for 1/2 hour.

## Claims

1. A compound of structural formula:

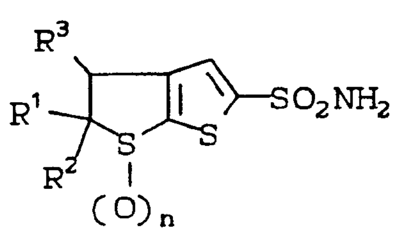

or

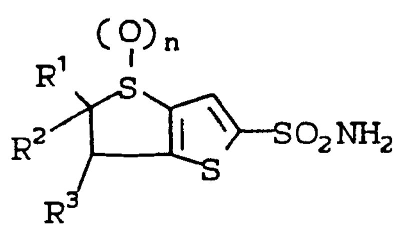

the individual diastereomers, the individual (R) or (S) enantiomer or mixtures thereof, or an ophthalmologically acceptable salt thereof wherein:
n is 0, 1 or 2, preferably 2;
$R^1$ is
1) hydrogen,
2) $C_{1-6}$alkyl, or
3) $C_{2-6}$alkenyl;
$R^2$ is $C_{1-6}$alkyl, either unsubstituted or substituted with
a) $C_{1-3}$alkyl-O-,
b) $C_{1-3}$alkyl-$S(O)_m$-,
c) $C_{1-3}$alkyl-O-$C_{1-3}$alkyl-O-
d) $C_{1-3}$alkyl-O-$C_{1-3}$alkyl-$S(O)_m$-,
e) $C_{1-3}$alkyl-$S(O)_m$-$C_{1-3}$alkyl-O-,
f) mono- or di-$C_{1-3}$alkyl-NH-,
g) $C_2$-$C_5$ alkenyl,
h) hydroxy,
i) hydroxy$C_{1-3}$alkyl-O-,
j) hydroxy$C_{1-3}$alkyl-$S(O)_m$-,
k) hydroxy$C_{1-3}$alkyl-O-$C_{1-3}$alkyl-O-,
l) hydroxy$C_{1-3}$alkyl-O-$C_{1-3}$alkyl-$S(O)_m$-,
m) hydroxymono- or di-$C_{1-3}$alkyl-NH-,
n) -COOH,
o) -C=N or
p) -$CONH_2$,
wherein m is 0, 1 or 2;
$R^3$ is hydroxy, amino, azido, $C_{1-6}$alkylamino, or $C_{3-6}$alkenylamino, with the alkyl group optionally substituted with hydroxy, F, $C_{1-3}$alkyl-O-, or $C_{1-3}$alkyl-$S(O)_m$-,
wherein m is 0, 1 or 2.

**2.** A compound of Claim 1 wherein $R^2$ is $C_{1-6}$alkyl, $C_{1-3}$alkoxy-$C_{1-6}$alkyl or $C_{1-3}$alkoxy-$C_{1-3}$-alkoxy$C_{1-3}$alkyl.

**3.** A compound of claim 2 wherein $R^2$ is $C_{1-6}$alkyl.

**4.** A compound of claim 2 wherein $R^2$ is $C_{1-3}$alkoxy-$C_{1-6}$alkyl.

**5.** A compound of claim 2 wherein $R^2$ is $C_{1-3}$alkoxy-$C_{1-3}$alkoxy-$C_{1-3}$alkyl.

**6.** A compound of any one of Claims 2 to 5 wherein $R^3$ is $C_{1-6}$alkylamino.

**7.** A compound of Claim 1 which is:
1) 4,5-dihydro-4-ethylamino-5-methyl-5-propyl-4H-thieno[2,3-b]thiophene-2-sulfonamide-6,6-dioxide;
2) 4,5-dihydro-5-methyl-5-propyl-4-propylamino-4H-thieno[2,3-b]thiophene-2-sulfonamide-6,6-dioxide;
3) 4,5-dihydro-4-ethylamino-4H-5-(2-methoxyethyl)-5-methylthieno[2,3-b]thiophene-2-sulfonamide-6,6-dioxide;
4) 4,5-dihydro-5-methyl-5-(2-methoxyethyl)-4-propylamino-4H-thieno[2,3-b]thiophene-2-sulfonamide;
5) 4,5-dihydro-5-methyl-5-(2-methoxyethyl)-4-propylamino-4H-thieno[2,3-b]thiophene-2-sulfonamide-6,6-dioxide;
6) 4,5-dihydro-4-ethylamino-4H-5-(3-methoxypropyl)-5-methylthieno[2,3-b]thiophene-2-sulfonamide-6,6-dioxide;
7) 4,5-Dihydro-4-hydroxy-5-propyl-4H-thieno[2,3-b]thiophene-2-sulfonamide-6,6-dioxide;
8) 4,5-Dihydro-4-azido-5-propyl-4H-thieno[2,3-b]thiophene-2-sulfnamide-6,6-dioxide;
9) 4,5-Dihydro-4-amino-5-propyl-4H-thieno[2,3-b]thiophene-2-sulfonamide-6,6-dioxide;
10) 4,5-Dihydro-4-(N-ethylamino)-5-propyl-4H-thieno[2,3-b]thiophene-2-sulfonamide-6,6-dioxide;
11) 4,5-Dihydro-4-(N-propylamino)-5-propyl-4H-thieno [2,3-b]thiophene-2-sulfonamide-6,6-dioxide
12) 4,5-Dihydro-4-azido-5-[2-(2-methoxyethoxy)ethyl]-4H-thieno[2,3-b]thiophene-2-sulfonamide-6,6-dioxide;
13) 4,5-Dihydro-4-amino-5-[2-(2-methoxyethoxy)ethyl]-4H-thieno[2,3-b]thiophene-2-sulfonamide-6,6-dioxide;

14) 4,5-Dihydro-4-(N-ethylamino)-5-[2-(2-methoxyethoxy)ethyl]-4H-thieno[2,3-b]thiophene-2-sulfonamide-6,6-dioxide; or
15) 4,5-Dihydro-4-hydroxy-5-[2-(2-methoxyethoxy)ethyl]-4H-thieno-[2,3-b]thiophene-2-sulfonamide-6,6-dioxide

**8.** The separated α and β isomers of4,5-Dihydro-4-(N-ethylamino)-5-[2-(2-methoxyethoxy)ethyl]-4H-thieno[2,3-b]thiophene-2-sulfonamide-6,6-dioxide.

**9.** The separated trans and cis isomers of 4,5-Dihydro-4-(N-ethylamino)-5-[2-(2-methoxyethoxy)ethyl]-4H-thieno[2,3-b]thiophene-2-sulfonamide-6,6-dioxide.

**10.** The separated (R,R), (S,S), (R,S), and (S,R) enantiomers of 4,5-Dihydro-4-(N-ethylamino)-5-[2-(2-methoxyethoxy)ethyl]-4H-thieno[2,3-b]thiophene-2-sulfonamide-6,6-dioxide.

**11.** An intermediate for a compound of claim 1 which is
1) 4,5-Dihydro-5-propyl-4-oxo-4H-thieno-[2,3-b]thiophene-6,6-dioxide
2) 4,5-Dihydro-4-hydroxy-5-propyl-4H-thieno-[2,3-b]thiophene-6,6-dioxide
3) 4,5-Dihydro-5-propyl-4-oxo-4H-thieno-[2,3-b]thiophene-6,6-dioxide
4) 4,5-Dihydro-4-hydroxyl-5-propyl-4H-thieno-[2,3-b]thiophene-6,6-dioxide
5) 4,5-Dihydro-4-oxo-5-[2-(2-methoxyethoxy)ethyl]-4H-thieno[2,3-b]thiophene-6,6-dioxide
6) Diethyl 2-(3-bromo-2-thienylthio)-2-[2-(2-methoxyethoxy)ethyl]malonate
7) 2-(3-Bromo-2-thienylthio)-4-(2-methoxyethoxy)-N-methyl-N-methoxybutyramide
8) 4,5-Dihydro-4-oxo-5-[2-(2-methoxyethoxy)ethyl]-4H-thieno[2,3-b]thiophene-6,6-dioxide
9) 4,5-Dihydro-4-hydroxy-5-[2-(2-methoxyethoxy)ethyl]-4H-thieno[2,3-b]thiophene-6,6-dioxide
10) 4,5-Dihydro-4-oxo-5-(2-methoxyethyl)-4H-thieno[2,3-b]thiophene-6,6-dioxide
11) 4,5-Dihydro-4-hydroxyl-5-(2-methoxy)ethyl-4H-thieno-[2,3-b]thiophene-6,6-dioxide
12) Ethyl 2-(3-bromo-2-thienylthio)acetate;
13) Ethyl 2-(3-bromo-2-thienylthio-4-methoxybutyrate;
14) 2-(3-Bromo-2-thienylthio)-4-methoxybutyric acid;
15) 2-(3-Bromo-2-thienylthio)-4-methoxy-N-methyl-N-methoxybutyramide;

**12.** An ophthalmological formulation comprising a pharmaceutically effective carrier and an effective amount of a compound of Claim 1.

**13.** The use of a compound of Claim 1 for the manufacture of a medicament for treating ocular hypertension.